(19) Europäisches Patentamt
European Patent Office
Office européen des brevets

(11) **EP 2 352 555 B1**

(12) **EUROPÄISCHE PATENTSCHRIFT**

(45) Veröffentlichungstag und Bekanntmachung des Hinweises auf die Patenterteilung:
**11.09.2013 Patentblatt 2013/37**

(21) Anmeldenummer: **09737348.4**

(22) Anmeldetag: **08.10.2009**

(51) Int Cl.:
***A61N 5/10*** *(2006.01)*

(86) Internationale Anmeldenummer:
**PCT/EP2009/007244**

(87) Internationale Veröffentlichungsnummer:
**WO 2010/043340 (22.04.2010 Gazette 2010/16)**

(54) **VORRICHTUNG UND VERFAHREN ZUR BESTIMMUNG VON STEUERPARAMETERN FÜR EINE BESTRAHLUNGSANLAGE**

DEVICE AND METHOD FOR DETERMINING CONTROL PARAMETERS FOR AN IRRADIATION UNIT

DISPOSITIF ET PROCÉDÉ POUR DÉTERMINER DES PARAMÈTRES DE COMMANDE POUR UNE INSTALLATION DE RADIOTHÉRAPIE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **13.10.2008 DE 102008051476**

(43) Veröffentlichungstag der Anmeldung:
**10.08.2011 Patentblatt 2011/32**

(73) Patentinhaber:
• **GSI Helmholtzzentrum für Schwerionenforschung GmbH
64291 Darmstadt (DE)**
• **Siemens Aktiengesellschaft
80333 München (DE)**

(72) Erfinder:
• **BERT, Christoph
63741 Aschaffenburg (DE)**

• **RIETZEL, Eike
64331 Weiterstadt (DE)**
• **GEMMEL, Alexander
91054 Erlangen (DE)**
• **SAITO, Nami
64291 Darmstadt (DE)**

(74) Vertreter: **Mergel, Volker
Blumbach - Zinngrebe
Patentanwälte
Alexandrastrasse 5
65187 Wiesbaden (DE)**

(56) Entgegenhaltungen:
**WO-A2-02/07817          WO-A2-2007/079854
US-A1- 2006 033 042**

**Beschreibung**

Die Erfindung betrifft eine Vorrichtung und ein Verfahren zur Bestimmung von Steuerparametern für eine Bestrahlungsanlage. Eine derartige Vorrichtung bzw. ein derartiges Verfahren findet insbesondere Einsatz im Rahmen der Partikeltherapie, beispielsweise im Rahmen der Therapieplanung, bei der im Vorfeld einer Bestrahlung Steuerparameter ermittelt werden, die es erlauben, anschließend während der Bestrahlung einen Gegenstand gemäß bestimmten Vorgaben zu bestrahlen. Weiterhin betrifft die Erfindung eine Bestrahlungsanlage und ein Bestrahlungsverfahren.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nichttherapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc. Hierbei werden geladene Partikel wie z.B. Protonen oder Kohlenstoffionen oder andere Ionen auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Objekt mit einem Zielvolumen mit dem Partikelstrahl bestrahlt.

Es kann dabei vorkommen, dass sich das zu bestrahlende Zielvolumen bewegt. Beispielsweise kann bei der Bestrahlung eines Patienten durch die Atembewegung eine Bewegung des zu bestrahlenden Tumors verursacht werden. Eine derartige Bewegung kann beispielsweise auch anhand von als Phantomen bezeichneten Modellobjekten für Forschungszwecke nachgebildet werden.

Insbesondere bei Bestrahlungsverfahren, bei denen eine Vielzahl von Bestrahlungsdosen sukzessive an unterschiedlichen Orten im Zielvolumen deponiert werden soll, ist es schwer, eine gewünschte homogene Dosisverteilung im Zielvolumen zu erreichen, wenn sich das Zielvolumen während des Fortgangs der Bestrahlung bewegt.

Bekannt sind einerseits Verfahren, bei denen das Zielvolumen lediglich zu bestimmten Zeitpunkten, an denen sich das Zielvolumen an einem bestimmten Ort oder in einer bestimmten Phase der Bewegung befindet, bestrahlt wird. Derartige Verfahren sind als Gating-Verfahren bekannt. Andererseits sind Verfahren bekannt, bei denen der Strahl der Bewegung des Zielvolumens nachgeführt wird. Derartige Verfahren sind unter dem Begriff Tracking-Verfahren bekannt.

Aus der US 6,891,177 B1, der US 6,710,362 B2 und der US 2006/0033042 A1 sind Verfahren und Vorrichtungen bekannt, mit denen eine Bewegungsnachführung des Strahls auch in Strahlrichtung durchgeführt werden kann. Auch die WO 02/07817 A2 und die WO 2007/079854 A2 zeigen derartige grundsätzliche Verfahren und Vorrichtungen.

Es ist die Aufgabe der Erfindung, eine Vorrichtung zur Bestimmung von Steuerparametern anzugeben, mit denen es möglich ist, eine Nachführung des Strahls vorteilhaft zu gestalten. Weiterhin ist es die Aufgabe der Erfindung, ein entsprechendes Verfahren zur Bestimmung von strahlparametern anzugeben.

Die Aufgabe wird durch die unabhängigen Ansprüche gelöst. Bevorzugte Ausgestaltungen der Erfindung sind in den abhängigen Ansprüchen angegeben und werden im Folgenden näher erläutert. Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Die erfindungsgemäße Vorrichtung zur Bestimmung von Steuerparametern für eine Bestrahlungsanlage, mit welcher eine Vielzahl von Strahlungsdosen nacheinander an unterschiedlichen Zielpunkten in einem Zielvolumen deponierbar ist, umfasst:

eine Eingabeeinrichtung, welche ausgebildet ist zum Erfassen von einem Zielgebiet und zum Erfassen einer Bewegung des Zielgebiets,
eine Auswertungseinrichtung zum Ermitteln von Steuerparametern zur Steuerung eines Strahls der Bestrahlungsanlage, derart, dass mit Hilfe der Steuerparameter ein Strahl der Bewegung des Zielgebiets nachführbar ist und in dem zielgebiet eine definierte Dosisverteilung deponierbar ist,
wobei die Auswertungseinrichtung dazu ausgelegt ist,

-   zur Ermittlung der Steuerparameter zumindest einen ersten wählbaren Steuerparameter zu ermitteln, sodass der Strahl der Bewegung des Zielgebiets lediglich orthogonal zur Strahlrichtung nachführbar ist, oder
-   zur Ermittlung der Steuerparameter zumindest einen ersten wählbaren Steuerparameter und einen weiteren, eine Energiemodulation der Strahls repräsentierenden, Steuerparameter zu ermitteln, wobei die Bewegungsnachführung in Strahlrichtung berücksichtigt ist.

Das Zielgebiet ist das zu bestrahlende Gebiet, etwa ein Tumor. Das zielvolumen umfasst vorzugsweise das Zielgebiet und entspricht dem Volumen, welches nach der Bestrahlungsplanung bestrahlt werde soll oder auch tatsächlich bestrahlt wird; das Zielvolumen kann insbesondere größer sein als das zielgebiet und etwa Sicherheitssäume umfassen.

Insgesamt "bestimmt" die Vorrichtung Steuerparameter für die Bestrahlungsanlage, dies kann etwa durch das

Angaben von relevante Steuerparameter umfassenden Zahlentupeln, jeweils für unterschiedliche Zeitpunkte, geschehen. Der konkrete Bestrahlungsablauf ist dabei, zumindest auch, eine Funktion der von der Vorrichtung bestimmten Steuerparameter. Die Auswertungseinheit "ermittelt" Steuerparameter, wobei die Wortwahl die Auslegung der Auswertungseinheit zum "Herausarbeiten" der Steuerparameter betont, etwa durch Berechnung. Natürlich können die Steuerparameter gegebenenfalls auch so weit ermittelt werden, dass diese bereits im oben dargestellten Sinne bestimmt sind; andererseits können auch andere Teile der Vorrichtung als die Auswertungseinheit zur Ermittlung beitragen. Die Unterscheidung ist also eher sprachlicher als technischer Natur; "Bestimmen" betont das Ergebnis, "Ermitteln" betont den Weg dahin.

Ein Steuerparameter ist "wählbar", wenn er nicht bereits festgelegt ist; etwa durch die physikalische Ausgestaltung der Bestrahlungsanlage. Gegebenenfalls kann eine Bedienerperson der Vorrichtung den zumindest einen ersten Steuerparameter (aus- ) wählen, so dass dann die Auswertungseinheit die zugehörigen Ausprägungen des Steuerparameters ermitteln kann. Das (Aus- ) wählen kann aber auch durch die Auswertungseinheit selbst, etwa durch Berechnung oder voreingestellt, erfolgen.

Die Bewegungsnachführung wird bei der Ermittlung der genannten Steuerparameter berücksichtigt; dies kann etwa durch die Bewegungsnachführung berücksichtigende Berechnungen erfolgen.

Es wurde erkannt, dass die zu applizierende, vorgegebene Dosisverteilung nicht nur mit einem einzigen Satz von Steuerparametern erreicht werden kann, sondern mit vielen, unterschiedlichen Sätzen von Steuerparametern. Die Steuerparameter umfassen folglich mehrere Freiheitsgrade, und bei der Ermittlung der Steuerparameter wird eine Wahl getroffen, die Anzahl gegebenenfalls verbliebener Freiheitsgrade wird dabei reduziert.

Die Bestimmung der Steuerparameter erfolgt unter Vorgabe von Randbedingungen zur Reduzierung der Freiheitsgrade. Eine erste Vorgabe stellt die (vorher)definierte Dosisverteilung selbst dar, die in dem Zielgebiet zu deponieren ist, gegebenenfalls unter Berücksichtigung von zu schonenden Strukturen (auch als OAR für "organ at risk" bekannt). Dies umfasst dabei auch, dass gegebenenfalls geringfügige Abweichungen von der vorgegebene Dosisverteilung toleriert werden können.

Eine weitere Vorgabe ist, dass der Strahl dem bewegten Zielgebiet zumindest in einer Richtung nachgeführt wird, dass die Dosisapplikation also unter Verwendung eines Tracking-Verfahrens erfolgt. Hierbei kann diese Richtung vorzugsweise orthogonal zur Strahlrichtung angeordnet sein.

Im Gegensatz dazu wird bei bisher bekannten Methoden ein Bestrahlungsplan für einen Referenzzustand festgelegt. Dieser Referenzzustand wird mit den unterschiedlichen Bewegungsphasen verglichen und Transformationsvorschriften zwischen dem Referenzzustand und den Bewegungsphasen erstellt. Diese Transformationsvorschriften können dann auf den Bestrahlungsplan angewendet werden. Die notwendige Strahlnachführung, auch in Strahlrichtung, errechnet sich aus diesen Transformationsvorschriften.

Dabei wurde erkannt, dass eine Bewegungsnachführung des Strahls in Strahlrichtung im Regelfall wesentlich aufwändiger durchzuführen ist als eine Strahlnachführung in Richtung orthogonal zur Strahlrichtung. Während Letzteres z.B. vergleichsweise einfach mithilfe von Scannermagneten erfolgen kann, erfordert Ersteres eine Energiemodulation des Strahls.

Daher kann nun die Auswertungseinrichtung derart ausgebildet sein, dass die Ermittlung der Steuerparameter so erfolgt, dass sich lediglich eine Bewegungsnachführung des Strahls in Richtung orthogonal zur Strahlrichtung ("laterales Tracking") ergibt. Die Ermittlung zumindest eines wählbaren Steuerparameters erfolgt also unter der Randbedingung bzw. Vorgabe, lediglich eine Bewegungsnachführung des Strahls in Richtung orthogonal zur Strahlrichtung ("laterales Tracking") zu verwenden.

Eine Alternativlösung kann aber auch darin bestehen, eine Bewegungsnachführung des Strahls in Strahlrichtung zuzulassen, die Steuerparameter aber derart zu bestimmen, dass die Bewegungsnachführung des Strahls in Strahlrichtung berücksichtigt wird. Dabei wird nicht nur derjenige Steuerparameter, welcher die Energiemodulation des Strahls repräsentiert - also diejenige Größe, die die Strahlnachführung in Strahlrichtung angibt - unter Berücksichtigung der Bewegungsnachführung des Strahls in Strahlrichtung bestimmt. Mindestens ein anderer Steuerparameter, vorzugsweise ein Steuerparameter, der nicht die Energie des Strahls betrifft, wird ebenso unter Berücksichtigung der Bewegungsnachführung des Strahls in Strahlrichtung bestimmt. Diese Steuerparameter betreffen beispielsweise die Einstrahlrichtung, die Anzahl der Felder einer Bestrahlung, die Gewichtung der Felder untereinander, die Fokusgröße des Partikelstrahls, einen Überlapp einzelner Zieldosen, eine zeitliche Abstimmung des Applikationszeitpunktes mit der Bewegung des Zielvolumens, den Scan-Pfad, die Scangeschwindigkeit, eine mögliche longitudinale Aufweitung des Strahls, die Anzahl der Bestrahlungssitzungen, eine mögliche zahl an Bestrahlungsdurchläufen je Bestrahlungssitzung, mehrere Teilbereiche des Zielvolumens und weitere und werden weiter unten erläutert. Wie diese Beispiele zeigen, brauchen entsprechende Steuerparameter sich also nicht unmittelbar auf die laterale Auslenkung, also auf ein laterales Scanning oder ein laterales Tracking, auszuwirken.

Insgesamt kann es so gesehen werden, dass bei der Bestimmung der Steuerparameter zu den Randbedingungen auch Randbedingungen gehören, welche die Bewegungsnachführung des Strahls in Strahlrichtung betreffen. Die Steuerparameter zur Strahlnachführung in Strahlrichtung werden also nicht nur lediglich nachträglich errechnet, die Strahlnachführung in Strahlrichtung fließt also in die Bestimmung der Steuerparameter von vornherein mit ein, auch für zumindest einen wählbaren Steuerparameter, der nicht unmittelbar mit der Energiemodulation des Strahls zusammenhängt oder unmittelbar mit der Strahlnachführung in lateraler Richtung.

Die freie Wählbarkeit eines Steuerparameters bedeutet, dass dieser Steuerparameter nicht als feste Größe bei der Ermittlung der Steuerparameter vorgegeben wird, sondern dass dieser Steuerparameter - gegebenenfalls innerhalb gewisser gesetzter Grenzen - variabel ist und bei der Bestimmung der Steuerparameter festgelegt wird.

Das Ziel, das Ausmaß der Bewegungsnachführung in Strahlrichtung zu verringern, kann also erreicht werden, indem bei der Ermittlung des zumindest einen wählbaren Steuerparameters die Bewegungsnachführung in Strahlrichtung berücksichtigt wird. Oder - alternativ - indem bei der Ermittlung der Steuerparameter lediglich ein laterales Tracking, also Tracking zur Strahlrichtung, zugelassen wird. In letzterem Falle ist die Bewegungsnachführung des Strahls in Strahlrichtung sozusagen "implizit" berücksichtigt, da eine derartige Strahlnachführung gar nicht zugelassen ist; eine entsprechende Ausgestaltung kann bereits durch die mechanische Ausgestaltung der Anlage bzw. und/oder auch der Vorrichtung vorgegeben sein.

In beiden Fällen gelingt es also, den zumindest einen wählbaren Steuerparameter so festzulegen, dass die aufwändigere Strahlnachführung in Strahlrichtung berücksichtigt und optimiert, insbesondere minimiert, wird. Dies bedeutet, dass der Bedarf oder das Ausmaß der Nachführung des Strahls in Strahlrichtung geringer ist als bei einer herkömmlichen Bestrahlungsplanung, also reduziert ist.

Das Zielgebiet kann dabei üblicherweise mithilfe eines Bilddatensatzes, in dem das Zielgebiet abgebildet ist, erfasst werden. Beispielsweise kann anhand eines CT-Datensatzes das Zielgebiet automatisch und/oder in Interaktion mit einem Anwender bestimmt werden. Ebenso können weitere Zielgebiete bestimmt werden, welche während einer nachfolgenden Bestrahlung zu schonen sind, d.h. mit einer möglichst geringen Dosis oder mit einer Dosis, welche auf jeden Fall unterhalb eines Grenzwertes liegen muss, belastet werden (so genannte OAR für engl: "organs at risk"). Auf diese weise kann eine Solldosisverteilung ermittelt werden, welche angibt, wie das Zielobjekt mit einer Bestrahlungsdosis belegt werden soll. Um diese Solldosisverteilung zu deponieren werden des weiteren Steuerparameter ermittelt.

Die Steuerparameter müssen dabei nicht zwangsläufig direkte Steuerparameter sein, d.h. Steuerparameter, welche unmittelbar - ohne weitere Interpretation oder Rechenschritte - zur Steuerung der Anlage eingesetzt werden können. Ein Bestrahlungsplan, welcher im Rahmen einer Bestrahlungsplanung entworfen wurde, kann auch einen Datensatz von Steuerparametern darstellen, der zur Bestimmung der Steuerparameter zur Steuerung der Anlage verwendet werden. Ein Bestrahlungsplan kann dann von der Bestrahlungsanlage geladen und zur Steuerung der Bestrahlungsanlage gemäß den im Bestrahlungsplan hinterlegten Vorgaben genutzt werden.

Die Steuerparameter, welche in einer ersten Phase während der Bestrahlungsplanung bestimmt werden, können dabei einen vollständigen Satz von Steuerparametern bilden. Dies bedeutet, dass der vollständige Satz von Steuerparametern ausreicht, um eine Bestrahlungsanlage zur Bestrahlung des Zielgebiets im Tracking-Verfahren zu steuern, insbesondere während der gesamten Bestrahlungszeit. Vollständigkeit betrifft sowohl die Anzahl der bestimmten Steuerparameter pro relevantem Zeitpunkt als auch die Anzahl der Zeitpunkte, für die einer der Steuerparameter bestimmt wird.

Dies ist jedoch nicht zwingend notwendig. Die Steuerparameter, welche in der ersten Phase bestimmt werden, können noch unvollständig sein. Dies bedeutet, dass der unvollständige Satz an Steuerparametern zwar schon für eine Bestrahlung des Zielgebiets im Tracking-Verfahren angelegt ist, wobei der unvollständige Satz aber erst in einer zweiten Phase durch weitere Steuerparameter ergänzt wird, sodass erst hierdurch ein vollständiger Satz von Steuerparametern entsteht. Es können also gegebenenfalls noch bisher nicht ermittelte Größen, etwa der Brennfleck oder Focus des Strahls, ermittelt werden, oder aber auch zu bereits ermittelten Größen, deren Ausprägung zu weiteren Zeitpunkten. Dies ermöglicht es auf einfache Weise, z.B. rechenintensive Kalkulationen während der ersten Phase im Rahmen der Bestrahlungsplanung zu implementieren, während in der zweite Phase, welche unmittelbar vor oder gar während der Bestrahlung stattfindet, die aktuelle Situation berücksichtigt werden kann. Dies ermöglicht sozusagen ein iteratives Nachfahren der Steuerparameter während der Bestrahlung.

Die Bewegung des Zielgebiets kann auch auf unterschiedliche Weise ermittelt werden. Beispielsweise kann die Bewegung des Zielgebiets aus einem Datensatz ermittelt werden, in welchem die Bewegung des Zielgebiets abgebildet ist, wie beispielsweise in einem 4D-CT-Datensatz.

In einer besonderen Ausgestaltung kann die Bewegung mithilfe bildgebender Verfahren auch am Behandlungsort direkt vor der Bestrahlung bestimmt werden. Die Bewegung kann weiterhin auch während einer stattfindenden Bestrahlung erfasst werden und dazu verwendet werden, Steuerparameter zum Steuern der Bestrahlungsanlage anzupassen oder zu ergänzen.

Es kann aber auch eine für das Zielgebiet typische, zu erwartende Bewegung verwendet werden. Wenn das zielgebiet beispielsweise eine Lunge ist, kann es je nach geforderter Genauigkeit und Regelmäßigkeit der Bewegung ausreichen, typische Bewegungen, die üblicherweise bei der Atmung auftreten, als Bewegung des Zielvolumens zu verwenden. Im Allgemeinen muss die Bewegung nicht explizit (z.B. als Amplitude über Zeit) angegeben werden. Es können auch Größen ausreichend sein, die sich aus der expliziten Bewegung ableiten lassen, wie beispielsweise Aufenthaltswahrscheinlichkeiten.

Außerdem ist es auch möglich, die Bewegung des Zielgebiets indirekt aufgrund anderer Bewegungen abzuleiten, d.h. beispielsweise kann anhand von Bewegungen an der Körperoberfläche auf die Bewegung des Zielgebiets geschlossen werden oder aber auch anhand der Bewegung anderer interner Strukturen auf die Bewegung des Zielgebiets geschlossen werden.

Neben der Bewegung des Zielgebiets kann auch die Bewegung anderer klinisch relevanter Gebiete erfasst werden, etwa wie die Bewegung eines zu schonenden Organs.

In vorteilhafter Weise ist die Auswertungseinrichtung derart ausgebildet, dass die Ermittlung der Steuerparameter derart erfolgt, dass der Strahl der Bewegung des Zielobjekts in zumindest zwei unterschiedlichen Richtungen nachgeführt wird.

Auf diese Weise kann ein Strahl in einer Ebene senkrecht zur Strahlverlaufsrichtung nachgeführt werden oder aber auch in allen drei Raumdimensionen.

In einer Ausführungsform ist die Auswertungseinrichtung derart ausgebildet, dass zur Ermittlung der Steuerparameter ein Maß bestimmt und genutzt wird, welches die durchzuführende Bewegungsnachführung in Strahlrichtung berücksichtigt.

Solch ein Maß bietet eine einfache Möglichkeit, die Bewegungsnachführung in Strahlrichtung bei der Bestimmung der Steuerparameter zu berücksichtigen. Beispielsweise kann das Maß in eine Zielfunktion einfließen, welche optimiert, d.h. z.B. minimiert, wird. Das Maß, das das Tracking in Strahlrichtung beschreibt, fließt dabei derart in die Zielfunktion ein, dass bestimmte Bewegungsnachführungen, etwa ein großes Ausmaß an Bewegungsnachführung, stärker oder weniger stark berücksichtigt wird als ein geringes Ausmaß an Bewegungsnachführung, was im Sinne der Erfindung als "bestraft" bezeichnet wird.

Die Berücksichtigung der Bewegungsnachführung in Strahlrichtung mit Hilfe des Maßes, worüber sich zum Beispiel das Ausmaß der Bewegungsnachführung als kontinuierlicher Wert erfassen lässt, ist jedoch nur eine Möglichkeit der Berücksichtigung. Beispielsweise kann auch eine Randbedingung vorgegeben werden, welche das maximale Ausmaß, das bei der Bewegungsnachführung in Strahlrichtung auftreten darf, begrenzt oder auf vorgegebene bzw. zu optimierende Werte diskretisiert. Auf diese Weise kann beispielsweise die Belastung eines Strahlnachführungssystems begrenzt werden, die bei der Durchführung der Bewegungsnachführung auftritt. Strahlparameter, die z.B. mithilfe einer Zielfunktion optimiert werden, können dabei, falls ein Scan-Verfahren eingesetzt wird, die Energie E des Strahls, der Focus F des Strahls, die Position x, y des Strahls in einer Ebene senkrecht zur Strahlrichtung, die Anzahl der Teilchen N an einem Rasterpunkt, die Änderung der Strahllage aufgrund der Bewegungsnachführung des Strahls dx, dy, dz in alle drei Raumrichtungen, der Scanpfad, die Scangeschwindigkeit (extrahierte Fluenz) und/oder die Änderung der Anzahl der Teilchen dN sein. Strahlparameter können dabei für jeden Rasterpunkt festgelegt werden.

Das Maß kann dabei beispielsweise eine Amplitude der Bewegungsnachführung des Strahls in Strahlrichtung, eine Geschwindigkeit der Bewegungsnachführung des Strahls in Strahlrichtung, und/oder eine Variation der Geschwindigkeit der Bewegungsnachführung des Strahls in Strahlrichtung berücksichtigen.

Das Maß kann dabei die von Rasterpunkt zu Rasterpunkt verwendete Amplitude, Geschwindigkeit und/oder Variation der Geschwindigkeit individuell für jeden Rasterpunkt berücksichtigen. Es ist aber auch denkbar, als Maß die über einen Bewegungszyklus auftretenden maximalen, mittleren, oder minimalen Werte zu verwenden, wodurch die Optimierung leichter zu implementieren ist; ggf. braucht das Maß nicht so oft ausgewertet zu werden.

In einem einfachen Fall können als Maß die benötigten Änderungen der Strahlreichweite $\delta z$ verwendet werden, welche in die Zielfunktion der Optimierung aufgenommen werden, und zwar so, dass große $\delta z$-Werte bestraft werden.

Wenn das Maß die Abfolge der Änderungen der $\delta z$-Werte über die Zeit angibt, können z.B. besonders "schnelle" Änderungen der $\delta z$-Werte bestraft werden, da diese besonders hohe Anforderungen an das Energiemodulationssystem stellen.

Das Maß kann aber auch eine weitere Größe beschreiben, die sich aus den $\delta z$-Werten ableiten lässt, z.B. eine daraus resultierende Anforderung an ein Energiemodulationssystem.

Das Maß kann auch dazu dienen, ein Abbruchkriterium für die Bestrahlung zu bestimmen, um eine Bestrahlung zu unterbrechen bzw. stoppen, falls die Bewegung extreme Werte annimmt, die eine sichere Bestrahlung ausschließt.

In einer Ausführungsform ist die Auswertungseinrichtung derart ausgebildet, dass durch den zumindest einen ersten Steuerparameter zumindest eine Bewegungsphase bestimmt wird, während der wenigstens ein erster Teil der Zielpunkte nicht angesteuert wird.

In dieser Ausführungsform kann nun als zusätzlicher Freiheitsgrad zumindest eine Bewegungsphase zugelassen sein. Mit dieser/n (wählbaren) Bewegungsphase/n kann beispielsweise ausgeschlossen werden, dass ein Teil der zielpunkte während einer Phase angesteuert wird, die ein großes Ausmaß an Strahlnachführung in Strahlrichtung bedingen würde, da sich z.B. in dieser Bewegungsphase das Zielgebiet besonders schnell bewegt. Der erste Teil der Zielpunkte kann auch alle Zielpunkte umfassen. In diesem Falle wird die Bestrahlung mit einem Gating-Verfahren kombiniert.

In einer anderen Ausführungsform ist die Auswertungseinrichtung derart ausgebildet, dass durch den zumindest einen ersten Steuerparameter wenigstens ein zweiter Teil der zielpunkte bestimmt wird, der nicht unter Verwendung einer Bewegungsnachführung des Strahls angesteuert wird.

In dieser Ausführungsform kann nun als Freiheitsgrad zugelassen werden, dass ein Teil der Zielpunkte festgelegt wird, der nicht "getrackt" werden soll. Durch die Festlegung derjenigen Zielpunkte, welche nicht unter Verwendung einer Bewegungsnachführung des Strahls angesteuert werden, kann beispielsweise bestimmt werden, dass für bestimmte, besonders kritische Zielpunkte keine Bewegungsnachführung durchgeführt werden soll. Beispielsweise können distale Schichten, die für einen Großteil der Dosis des Zielgebiets verantwortlich sind, lediglich mit einem Gating-Verfahren ohne Tracking bestrahlt werden. Dies kann der Sicherstellung der Dosimetrie dienen. Der Rest des Feldes, in dem insgesamt vergleichsweise weniger Dosis direkt deponiert wird, kann dann z.B. lediglich mit lateralem Tracking bestrahlt werden. Dies kann dann immer noch ausreichen, um eine gewünschte Dosisverteilung in ausreichender Genauigkeit zu deponieren, auch wenn eine Bewegungsnachführung in Strahlrichtung nun nicht durchgeführt wird. Die beschriebene Ausprägung des Verfahrens zielt dabei dann auch auf eine Optimierung der Bestrahlungsparameter ab, die einzelne Bereiche des Zielvolumens betrifft.

In einer anderen Ausführungsform ist die Auswertungseinrichtung derart ausgebildet, dass durch den zumindest einen ersten Steuerparameter ein wiederholtes Anfahren der Rasterpunkte und/oder eine Reihenfolge des Anfahrens der Zielpunkte bestimmt werden.

In dieser Ausführungsform kann als Freiheitsgrad zugelassen werden, dass Zielpunkte wiederholt angefahren werden - also ein sogenanntes Rescanning durchgeführt werden kann - oder aber auch der Strahlpfad, d.h. die Reihenfolge des Anfahrens der Zielpunkte, variabel gestaltet werden kann.

Ohne Rescanning kann es zum Beispiel nicht möglich sein, eine bestimmte Homogenität der Dosisverteilung zu erreichen und gleichzeitig eine bestimmte Vorgabe bezüglich des Tracking in Strahlrichtung zu erfüllen, da Rescanning über Mittelungseffekte die Homogenität der Bestrahlung fördert. Wenn nun aber als Freiheitsgrad ein Rescanning zugelassen wird, kann es möglich werden, die Homogenität der Dosisverteilung zu erreichen und gleichzeitig die Vorgabe bezüglich des Tracking in Strahlrichtung zu erfüllen. Selbiges gilt für den Strahlpfad. Wenn ein bestimmter Strahlpfad es nicht erlaubt, eine Vorgabe bezüglich des Tracking in Strahlrichtung zu erfüllen, kann eine Änderung des Strahlpfades bereits ausreichen, dass die Vorgabe erfüllt wird.

In einer anderen Ausführungsform ist die Auswertungseinrichtung derart ausgebildet, dass durch den zumindest einen ersten Steuerparameter eine räumliche Orientierung einer Einstrahlrichtung, eine Anzahl von Einstrahlrichtungen und/oder ein Überlapp von Rasterpunkten ermittelt wird.

Auch diese Parameter können als Freiheitsgrad in die Ermittlung der Steuerparameter einfließen. So kann bereits eine Änderung der Einstrahlrichtung ausreichen, eine bestimmte Vorgabe bezüglich des Tracking in Strahlrichtung zu erfüllen. Dies kann etwa auch erreicht werden, indem die Dosis auf verschiedene Felder aufgeteilt wird, welche gegebenenfalls unterschiedlich gewichtet werden. Auch eine Änderung des Überlapps der Zielpunkte sowohl in lateraler als auch longitudinaler Richtung kann eine gewünschte Homogenität der Dosis unter Berücksichtigung der Vorgabe wiederherstellen. Ein Überlapp ist gegeben durch den Abstand der Zielpunkte zueinander und durch die Fokusgröße des Strahls bei der Bestrahlung der Zielpunkte.

Es können auch eine oder mehrere Einstrahlrichtungen gewählt werden. Diese Einstrahlrichtungen geben an, aus welcher Richtung bezogen auf das Zielobjekt der Partikelstrahl auf das Zielobjekt gerichtet wird. In einer Anlage lässt sich dies beispielsweise derart umsetzen, dass das Zielobjekt zum Strahl entsprechend positioniert wird, und/oder dass die räumliche Orientierung des Strahls - beispielsweise über eine entsprechende Position einer Gantry - eingestellt wird.

Eine erfindungsgemäße Bestrahlungsanlage, insbesondere Partikeltherapieanlage, weist eine Vorrichtung zur Bestimmung von Steuerparametern nach einem der Ansprüche 1 bis 9 auf.

Hierdurch ist es möglich, ein Energiemodulationssystem, welches zur Bewegungsnachführung eines Strahls in Strahlrichtung eingesetzt wird, einfacher und damit kostengünstiger auszugestalten oder gar die Anlage ohne ein Energiemodulationssystem einzusetzen. Durch die Bestimmung der Steuerparameter können Vorgaben hinsichtlich einer Anforderung an das Energiemodulationssystem oder an ein Tracking in Strahlrichtung, welches bei einem lediglich lateralen Tracking nicht durchgeführt wird, berücksichtigt werden.

Das erfindungsgemäße Verfahren zum Bestimmen von Steuerparametern für eine Bestrahlungsanlage, mit welcher eine Vielzahl von Strahlungsdosen nacheinander an unterschiedlichen Zielpunkten in einem Zielvolumen deponiert werden kann, weist die folgenden Schritte auf: Erfassen eines Zielgebiets mit einer Eingabeeinrichtung, Erfassen einer Bewegung des Zielgebiets mit der Eingabeeinrichtung, Ermitteln von Steuerparametern zur Steuerung eines Strahls der Bestrahlungsanlage mit einer Auswertungseinrichtung, derart, dass mit den Steuerparametern der Strahl der Bewegung des Zielgebiets nachgeführt werden und in dem Zielgebiet eine definierte Dosisverteilung deponiert werden kann, wobei bei der Ermittlung der Steuerparameter durch die Auswertungseinrichtung zumindest ein erster wählbarer Steuerparameter derart ermittelt wird, dass der Strahl der Bewegung des Zielgebiets lediglich orthogonal zur Strahlrichtung nachgeführt wird, oder wobei bei der Ermittlung der Steuerparameter durch die Auswertungseinrichtung zumindest ein erster wählbarer Steuerparameter und ein weiterer, eine Energiemodulation des Strahls repräsentierender Steuerparameter ermittelt werden, wobei die Bewegungsnachführung in Strahlrichtung berücksichtigt wird.

Der weitere Steuerparameter, der eine Energiemodulation des Strahls in Strahlrichtung repräsentiert, steht also in unmittelbarem Zusammenhang mit der Strahlnachführung in Strahlrichtung. Der weitere Steuerparameter muss also zwangsläufig unter Berücksichtigung der Bewegungsnachführung in Strahlrichtung bestimmt werden. Nun wird jedoch auch noch ein anderer Steuerparameter, also der erste wählbare Steuerparameter, unter Berücksichtigung der Bewegungsnachführung in Strahlrichtung bestimmt. Dieser Steuerparameter muss nicht im unmittelbaren Zusammenhang mit der Energiemodulation des Strahls stehen, welche für eine Bewegungsnachführung des Strahls in Strahlrichtung notwendig ist. Anders ausgedrückt, dieser Steuerparameter beeinflusst zumindest einen von der Energiemodulation unabhängigen Aspekt der Bestrahlung. Weiter braucht dieser erste wählbare Steuerparameter auch nicht unmittelbar die laterale Auslenkung des Strahls zu beeinflussen, wie weiter vorne bereits ausgeführt; er kann etwa auf eine Drehung der Gantry wirken. Er kann insbesondere unabhängig von lateralem Scanning und lateraler Nachführung sein.

Ähnliches gilt für den ersten wählbaren Steuerparameter, der so ermittelt wird, dass der Strahl der Bewegung des Zielgebiets lediglich orthogonal zur Strahlrichtung nachgeführt wird. Auch dieser Parameter braucht nicht selbst in unmittelbarem Zusammenhang mit der Strahlnachführung in orthogonaler Richtung oder lateralem Scanning zu stehen. Anders ausgedrückt, dieser Steuerparameter kann einen von der Strahlnachführung in orthogonaler Richtung oder lateralem Scanning unabhängigen Aspekt der Bestrahlung beeinflussen.

Weiterhin wird ein Verfahren beschrieben zur Bestrahlung eines bewegten Zielgebiets mit einem Satz von Steuerparametern zur Steuerung einer Bestrahlungseinrichtung, welche Steuerparameter nach dem oben dargestellten Verfahren bestimmt worden sind.

Insbesondere kann mit dem Verfahren zur Bestrahlung ein Zielgebiet bestrahlt werden, das ein Teil eines nicht-lebenden Körpers ist, z.B. innerhalb eines Phantoms, welches zur Überprüfung einer Bestrahlungsplanung, zu Forschungszwecken an beispielsweise Zellkulturen eingesetzt wird.

Grundsätzlich betrifft die Erfindung auch eine Vorrichtung zur Bestimmung von Steuerparametern für eine Bestrahlungsanlage, mit welcher eine Vielzahl von Strahlungsdosen nacheinander an unterschiedlichen Zielpunkten in einem Zielvolumen deponierbar ist, umfassend:

eine Eingabeeinrichtung, welche ausgebildet ist zum Erfassen von einem Zielgebiet und zum Erfassen einer Bewegung des Zielgebiets,
eine Auswertungseinrichtung zum Ermitteln von Steuerparametern zur Steuerung eines Strahls der Bestrahlungsanlage, derart, dass mit Hilfe der Steuerparameter ein Strahl der Bewegung des Zielgebiets nachführbar ist und in dem Zielgebiet eine definierte Dosisverteilung deponierbar ist,
wobei die Auswertungseinrichtung dazu ausgelegt ist,
zur Ermittlung der Steuerparameter zumindest einen ersten wählbaren Steuerparameter und einen weiteren, eine Energiemodulation des Strahls repräsentierenden, Steuerparameter zu ermitteln, wobei die Bewegungsnachführung in Strahlrichtung dadurch berücksichtigt ist, dass die Energiemodulation des Strahls unterdrückt ist und der Strahl der Bewegung des Zielgebiets lediglich orthogonal zur Strahlrichtung nachführbar ist.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:

Figur 1 einen schematischen Überblick über den Aufbau einer Partikeltherapieanlage,

Figur 2 eine schematische Darstellung eines mittels einer Rasterscaneinrichtung zu bestrahlenden Zielvolumens,

Figur 3 ein Ablaufdiagramm eines Verfahrens zur Bestimmung von Steuerparametern für die in Figur 1 gezeigte Bestrahlungsanlage,

Figur 4 eine Übersicht über verschiedene Steuerparameter, welche unter Berücksichtigung der Bewegungsnachführung des Strahls in Strahlrichtung ermittelt werden, und

Figur 5 ein Ablaufdiagramm für die Bestimmung von Steuerparametern mit Hilfe einer Zielfunktion.

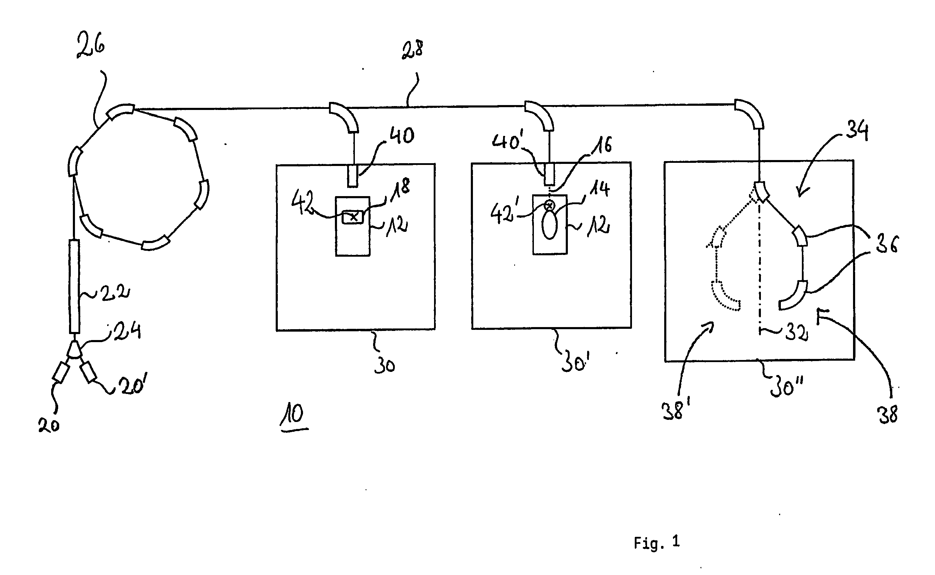

Figur 1 zeigt in schematischer Darstellung einen Aufbau einer Partikeltherapieanlage 10. Die Partikeltherapieanlage 10 wird zur Bestrahlung eines auf einer Positioniervorrichtung 12 angeordneten Körpers 14 mit einem Strahl aus Partikeln 16 eingesetzt, der im Folgenden als Partikelstrahl 16 bezeichnet ist. Insbesondere wird ein tumorerkranktes Gewebe eines Patienten mit dem Partikelstrahl bestrahlt. Es ist ebenfalls vorgesehen, die Partikelstrahlanlage 10 zur Bestrahlung eines nicht-lebenden Körpers 18, insbesondere eines Wasserphantoms 18 einzusetzen. Die Bestrahlung des Wasserphantoms 18 erfolgt beispielsweise zu Zwecken der Überprüfung und Verifizierung von Bestrahlungsparametern vor und/oder nach einer erfolgten Bestrahlung eines Patienten 14. Es ist aber auch vorgesehen, andere Körper, insbesondere Versuchsaufbauten wie beispielsweise Zellkulturen oder Bakterienkulturen zu Forschungszwecken mit dem Partikelstrahl 16 zu bestrahlen. In allen Fällen kann es sich um bewegte oder ruhende Körper 14, 18 handeln.

Als Partikel werden vornehmlich Teilchen wie beispielsweise Protonen, Pionen, Heliumionen, Kohlenstoffionen oder Ionen anderer Elemente eingesetzt. Üblicherweise werden derartige Partikel in einer Partikelquelle 20, die im Folgenden als Ionenquelle 20 bezeichnet wird, erzeugt. Wenn, wie dies in Figur 1 dargestellt ist, die Partikelstrahlanlage 10 zwei Partikelquellen, z.B. zwei Ionenquellen 20 und 20' aufweist, ist zwischen den Ionenquellen 20 und 20' und einem Vorbeschleuniger 22 ein Schaltmagnet 24 angeordnet. Mittels des Schaltmagneten 24 kann sowohl der von der Ionenquelle 20 und der Ionenquelle 20' erzeugte Ionenstrahl in den Vorbeschleuniger 22 eingespeist werden, wobei innerhalb eines kurzen Zeitintervalls der Ionenstrahl aus der Ionenquelle 20 und der Ionenquelle 20' abwechselnd eingespeist werden können. Es wird zwischen den Ionenquellen 20 und 20' umgeschaltet. Auf diese Weise können abwechselnd oder innerhalb eines kurzen Zeitintervalls hintereinander Partikelstrahlen mit zwei unterschiedlichen Ionensorten in der Partikeltherapieanlage 10 eingesetzt werden. Beispielsweise können Partikelstrahlen mit Protonen und mit Kohlenstoffionen nahezu gleichzeitig betrieben werden, indem in sehr kurzen Zeitintervallen zwischen den Ionenquellen 20 und 20' geschaltet wird, wobei hierbei beispielsweise die Ionenquelle 20 einen Protonenstrahl und die Ionenquelle 20' einen Kohlenstoffionenstrahl erzeugt.

Der von der oder einer der Ionenquellen 20, 20' erzeugte und gegebenenfalls mit dem Schaltmagneten 24 ausgewählte Ionenstrahl oder Partikelstrahl wird in dem vorbeschleuniger 22 auf ein erstes Energieniveau beschleunigt. Der Vorbeschleuniger 22 ist beispielsweise ein Linearbeschleuniger (LINAC für engl.: "LINear ACcelerator"). Anschließend werden die Partikel in einen weiteren Beschleuniger 26, beispielsweise einen Kreisbeschleuniger, insbesondere ein Synchrotron oder zyklotron, eingespeist. In dem Beschleuniger 26 wird der Partikelstrahl auf mindestens eine zur Bestrahlung eines in einem Körper 18 abgeordneten Zielvolumen (nicht dargestellt) benötigte Energie beschleunigt. Nachdem der Partikelstrahl den Beschleuniger 26 verlassen hat, transportiert ein HochenergiestrahlTransportsystem 28 den Partikelstrahl in einen oder mehrere Bestrahlungsräumen 30, 30', 30", wobei dort beispielsweise die Positioniervorrichtung 12 - etwa eine Patientenliege - mit dem Patienten 14 oder das Phantom 18 zur Bestrahlungsplanungsverifikation angeordnet ist. In dem Bestrahlungsraum 30 oder 30' erfolgt die Bestrahlung des Körpers 14, 18 von einer festen Richtung aus, wobei der Körper 14, 18 raumfest angeordnet ist. Diese Bestrahlungsräume 30, 30' werden als "fixed beam"-Räume bezeichnet. Im Behandlungsraum 30" ist eine um eine Achse 32 beweglich angeordnete, vorzugsweise drehbar angeordnete Gantry 34 vorgesehen. Mittels der Gantry 34 kann der zu bestrahlende Körper 14 oder das Phantom 18 von verschiedenen Richtungen aus bestrahlt werden. Hierbei wird der Partikelstrahl mittels der in der Gantry 34 angeordneten Gantrystrahlführung 36 um den zu bestrahlenden Körper 14, 18 gedreht. Es sind in Figur 1 stellvertretend für die unterschiedlichen Positionen der Gantrystrahlführung 36 der Gantry 34 eine erste Position 38 und eine zweite Position 38' gezeigt. Selbstverständlich sind auch Zwischenpositionen für die Gantrystrahlführung 36, die aus Gründen der Übersichtlichkeit nicht gezeigt sind, auf zumindest einer Halbkugel oberhalb des zu bestrahlenden Körpers 14, 18 in einer gedachten Kugel um den zu beststrahlenden Körper 14, 18 möglich. Somit kann das zu bestrahlende Zielvolumen von mehreren Richtungen aus senkrecht zur Achse 32 bestrahlt werden. Dies ist aus geometrischen Gründen vorteilhaft.

Im Bestrahlungsraum 30, 30' tritt der Partikelstrahl aus einem als Strahlauslass 40, 40' bezeichneten Ende eines Vakuumsystems der Hochenergiestrahlführung 28 aus und trifft auf das zu bestrahlende Zielvolumen (nicht dargestellt) im Körper 14 oder 18. Das Zielvolumen ist hierbei üblicherweise in einem Isozentrum 42, 42' des jeweiligen Bestrahlungsraums 30, 30' angeordnet.

Der anhand der Figur 1 dargestellte Grundaufbau einer Partikeltherapieanlage 10 ist beispielhaft für Partikeltherapieanlagen, kann aber auch hiervon abweichen.

Die nachfolgend beschriebenen Ausführungsbeispiele sind sowohl im Zusammenhang mit der anhand von Figur 1 dargestellten Partikeltherapieanlage als auch mit anderen Partikeltherapieanlagen einsetzbar.

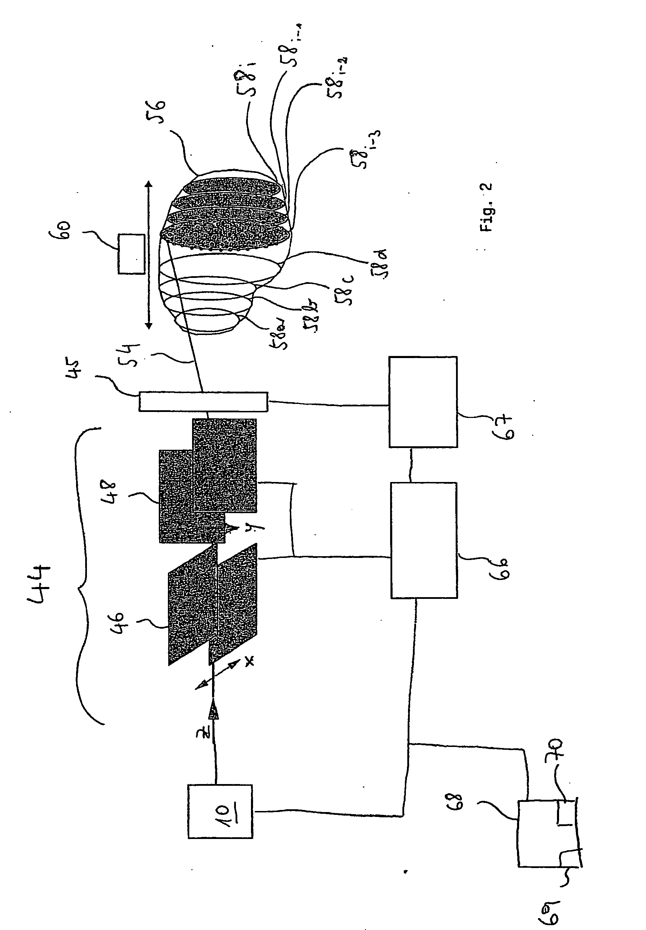

Figur 2 zeigt eine schematische Darstellung von Einrichtungen, die für eine Bestrahlung im Sinne der Erfindung verwendet werden können, hierbei ist insbesondere eine Rasterscaneinrichtung 44 und eine Energiemodulationseinrichtung 45 schematisch dargestellt.

Gleiche Bezugszeichen bezeichnen hierbei gleiche Gegenstände. Die Rasterscaneinrichtung 44 weist eine erste Partikelstrahlablenkungseinrichtung 46 und eine zweite Partikelstrahlablenkungseinrichtung 48 auf, welche insbesondere Magneten umfassen können. Die beiden Partikelstrahlablenkeinrichtungen 46, 48 können den Strahl horizontal bzw. vertikal ablenken. Der Pfeil 50 zeigt die Ablenkrichtung eines Partikelstrahls 54 in x-Richtung (horizontal) und der Pfeil 52 zeigt die Ablenkung des Partikelstrahls 54 in y-Richtung (vertikal). Somit ist mittels der Rasterscanneinrichtung 44 der Partikelstrahl 54 in der Lage, eine Matrix aus Punkten mit den Positionen $(x_j, y_j)$ zu scannen oder abzufahren, wobei i die Anzahl der abzufahrenden Punkte der Matrix ist. Diese Punkte $(x_j, y_j)$ werden als Rasterpunkte bezeichnet. Das zu bestrahlende Zielvolumen 56 in dem Körper 14 oder 18 setzt sich aus isoenergetischen Scheiben oder Schichten 58a, 58b, 58c, ... 58i mit jeweils unterschiedlichen Feldern von Rasterpunkten $(x_j, y_j)$ zusammen. Hierbei sind die iosenergetischen Schichten 58a, 58b, 58c, ... 58i jeweils einer bestimmten Position auf der z-Achse zugeordnet.

In dem hier dargestellten Beispiel beginnt die Zählung der Schichten an der Rasterscaneinrichtung 44 zugewandten Seite mit 58a, während die von der Rasterscaneinrichtung 44 am weitesten entfernte Schicht (distale Schicht) die Bezeichnung 58i hat, wobei i die Anzahl der Schichten bezeichnet. Zur Einstellung des Partikelstrahls 54 auf eine jeweiligen Schicht 58a, 58b, 58c, ... 58i weist der Partikelstrahl 54 jeweils eine andere Energie auf. Hierbei wird der Partikelstrahl 54 mit der niedrigsten Energie in der Scheibe 58a und der Partikelstrahl 54 mit der höchsten Energie in der Scheibe 58i deponiert.

Die Bestrahlung mit einem Scan-Verfahren umfasst folglich einen Partikelstrahl 54, welcher so dimensioniert ist, dass in dem Zielvolumen 56 lediglich an einem kleinen, umschriebenen Bezirk eine Einzeldosis deponiert werden kann. Ein solcher kleiner Bezirk kann folglich einem Rasterpunkt zugeordnet werden, wobei die Parameter der Rasterpunkte - also die Koordinaten der Rasterpunkte und/oder die Parameter des Partikelstrahls, die auf die Koordinaten der Rasterpunkte abgestimmt sind - vorzugsweise in der Bestrahlungsplanung enthalten sind.

Um das gesamte Zielvolumen 56 zu bestrahlen, werden sukzessive unterschiedliche Rasterpunkte, die Orte des Zielvolumens 56 sind, nacheinander bestrahlt. Der Partikelstrahl 54 wird dabei mithilfe von Scan-Magneten 46 und 48 abgelenkt und so über das Zielvolumen gescannt, wodurch die Rasterpunkte abgescannt oder abgetastet werden. Zur Bestrahlung unterschiedlicher Iso-Energieschichten wird die Energie des Partikelstrahls 54 passend eingestellt. Gezeigt ist ein Zielvolumen 56, bei dem drei distale Iso-Energieschichten $58_i$, $58_{i-i}$, $58_{i-2}$ bereits bestrahlt worden sind, und bei denen der Partikelstrahl 54 über die nachfolgenden Iso-Energieschicht $58_{i-3}$ scannt.

Es sind verschiedene Scan-Verfahren bekannt, wie z.B. das Raster-Scanning, bei dem der Strahl ohne Abschaltung zwischen benachbarten Rasterpunkten über das Zielvolumen scannt, das Spot-Scanning, bei dem eine Abschaltung zwischen Zielpunkten erfolgt, oder kontinuierliche Scan-Verfahren, bei denen der Strahl kontinuierlich abgelenkt wird.

Gegebenenfalls kann noch eine Energiemodulationseinrichtung 45 vorgesehen sein. Diese kann beispielsweise zwischen den Scan-Magneten 46, 48 und dem Zielvolumen 56 angeordnet sein, wobei mit dieser die Eindringtiefe des Partikelstrahls 54 einer Bewegung des Zielvolumens 56 nachgeregelt werden kann. Die Energiemodulationsvorrichtung kann z.B. wie in einer der Schriften US 6,891,177 B1, US 6,710,362 B2 oder US 2006/0033042 A1 beschrieben ausgebildet sein.

Es kann zusätzlich und/oder alternativ auch eine Energiemodulationseinrichtung - in Strahlrichtung gesehen - vor den Scan-Magneten 46, 48 vorgesehen sein, welche zur Bewegungsnachführung des Partikelstrahls in Strahlrichtung ausgebildet ist. Diese Energiemodulationseinrichtung kann als selbständige Einheit analog zu 45 ausgebildet sein oder aber eine Eigenschaft des Beschleunigers darstellen. Im letzteren Fall ist der Beschleuniger in der Lage, die Strahlenergie in Zeitintervallen, die der Bestrahlungsdauer eines Rasterpunktes entsprechen, zu verändern.

Eine Energiemodulationsvorrichtung 45 kann auch für ein sogenanntes Tiefen-Scanning verwendet werden. Dies bedeutet, dass nicht die Schichten nacheinander bestrahlt werden, sondern dass der Scanpfad auch zwischen den Schichten verlaufen kann.

Eine derartige Energiemodulationseinrichtung 45, mit der eine Bewegungsnachführung in Strahlrichtung möglich ist, muss aber nicht vorhanden sein. In diesem Fall wird die Bestrahlungsplanung bzw. die Ermittlung von Steuerparametern derart gestaltet, dass eine Bestrahlung des bewegten Zielvolumens 56 lediglich mit einer Bewegungsnachführung in einer Richtung orthogonal zur Strahlrichtung durchgeführt wird.

Alternativ und/oder zusätzlich kann eine - in Strahlrichtung 16 gesehen - vor den Scan-Magneten 46, 48 angeordnete Energiemodulationsvorrichtung verwendet werden, die jeweils die Energien des Partikelstrahls 54 für die verschiedenen Iso-Energieschichten einstellt. Letzteres Verfahren wird insbesondere bei Partikelstrahlanlagen mit einem Zyklotron angewendet.

Durch den Scan-Prozess entsteht folglich eine zeitlich protrahierte Bestrahlung, während der die zu deponierende Dosis inkrementell deponiert wird. Diese Dosis erzeugt eine inkrementell anwachsende Aktivitätsverteilung.

Die Positionierung des Partikelstrahls 54 im Zielvolumen 56 kann z.B. mittels einer Positronenemissionstompographie-Einrichtung (PET) überprüft werden. Die PET-Einrichtung weist mindestens zwei Detektoren und eine Steuereinrichtung auf, die in Wirkverbindung mit der Steuereinrichtung der Rasterscaneinrichtung 44 steht, wodurch die Datenaufzeichnung mit der PET-Einrichtung und die Bestrahlung aufeinander abgestimmt werden können.

Vor der Bestrahlung eines Zielvolumens 56 wird eine Bestrahlungsplanung durchgeführt, um dann die Bestrahlung, d.h. das Scannen des Zielvolumens 56 mit dem Partikelstrahl 54, entsprechend der erstellten Bestrahlungsplanung zu steuern. Die Bestrahlungsplanung stellt also die Bestimmung von Steuerparametern zur Steuerung der Bestrahlungsanlage 10 dar. Die Bestrahlungsplanung wird mit einer eigens dafür ausgebildeten Bestrahlungsplanungsvorrichtung 68 durchgeführt.

Hierzu wird mittels eines Computer-Tomographen oder Kernspin-Tomographen oder mittels anderer diagnostischer Einrichtungen die Lage und Ausdehnung eines zu bestrahlenden Tumors oder eines anderen Zielvolumens 56 ermittelt.

Neben der Lage und Ausdehnung eines zu bestrahlenden Zielvolumens 56 wird auch die Bewegung des zielvolumens 56 und gegebenenfalls anderer klinisch relevanter Volumina ermittelt. Dies kann auf verschiedene Art und Weise geschehen. Zum einen kann die Bildgebungsmodalität derart ausgebildet sein, dass durch die Bildgebung selbst die Bewegung des Zielvolumens erfasst wird, wie beispielsweise bei einer 4D-Computertomographie. Die Bewegung des Zielvolumens 56 kann mithilfe eines Bewegungserfassungssystems 60 erfasst werden.

Verschiedene Bewegungserfassungssysteme 60 können eingesetzt werden: Z.B. kann die Bewegung der Bauchdecke mit Hilfe der Messung einer Bewegungsamplitude eines Infrarotmarkers mit einem Kamerasystems detektiert werden. Es ist auch möglich, die Bewegungsphase aus der Dehnung eines um den Bauch oder den Brustkorb geschnallten Sensors zu ermitteln. Wird das Verfahren der Spirometrie verwendet, atmet der Patient durch einen Volumensensor, mit dem zeitlich aufgelöst das Volumen der ein- und ausgeatmeten Luft bestimmt werden kann. Auch ein Temperatursensor, der über den Verlauf der Atmung Auskunft geben kann, ist vorgesehen. Die Bewegungserfassung kann auch mit Hilfe eines kleinen, insbesondere reiskorngroßen elektromagnetischen Transponders, welcher in dem Zielvolumen implantiert ist, erfolgen. Auch externe Bildgebungssysteme, wie z.B. Ultraschall oder Fluoroskopie, können zur Überwachung der Bewegung des Zielvolumens eingesetzt werden, wobei beispielsweise implantierte Marker verwendet werden und die Erfassung der Bewegung unterstützen, indem diese mit dem Bildgebungssystem detektiert werden können. Prinzipiell können auch Systeme, die eine volumetrische Bildgebung ermöglichen, eingesetzt werden.

Je nach Regelmäßigkeit der zu erwartenden Bewegung kann es auch lediglich ausreichen, die zu erwartende Bewegung des Zielvolumens 56 anzugeben. In diesem Fall gibt die zu erwartende Bewegung die tatsächliche Bewegung des Zielvolumens 56 an. Eine gezielte Überwachung des Zielvolumens 56 hinsichtlich der tatsächlichen Bewegung ist nicht notwendig.

Daten der Bildgebungsvorrichtung werden unmittelbar oder nach einer Aufbereitung durch weitere, nicht dargestellte Einrichtungen, der Bestrahlungsplanungsvorrichtung 68 über eine Eingabeeinrichtung 69 der Bestrahlungsplanungsvorrichtung 68 bereitgestellt. Die Bestrahlungsplanungsvorrichtung 68 erstellt einen Datensatz, welcher die Steuerparameter umfasst, die zur Steuerung der Bestrahlungsanlage gemäß bestimmter Vorgaben eingesetzt werden. Die Bestrahlungsplanungsvorrichtung 68 ist etwa ein Arbeitsplatz-Computer, eine Workstation oder ein anderer Rechner. Üblicherweise ist die Bestrahlungsplanungsvorrichtung 68 ferner durch ihre Benutzerschnittstelle, Software oder andere Merkmale dazu ausgebildet, dass ein Anwender mit der Vorrichtung 68 das oder die Zielvolumen 56, die zu applizierende Dosisverteilung, die Aufteilung derselben auf mehrere Durchgänge, die Richtung der Bestrahlung und andere Details, welche die Bestrahlungsplanung betreffen, definiert.

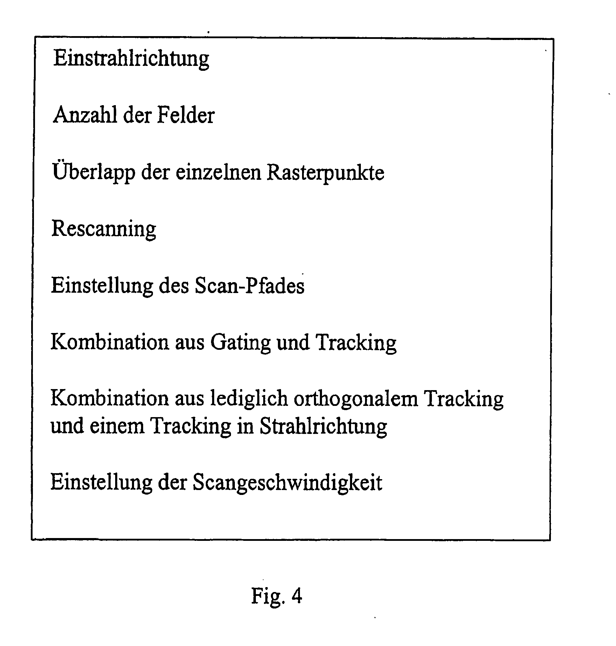

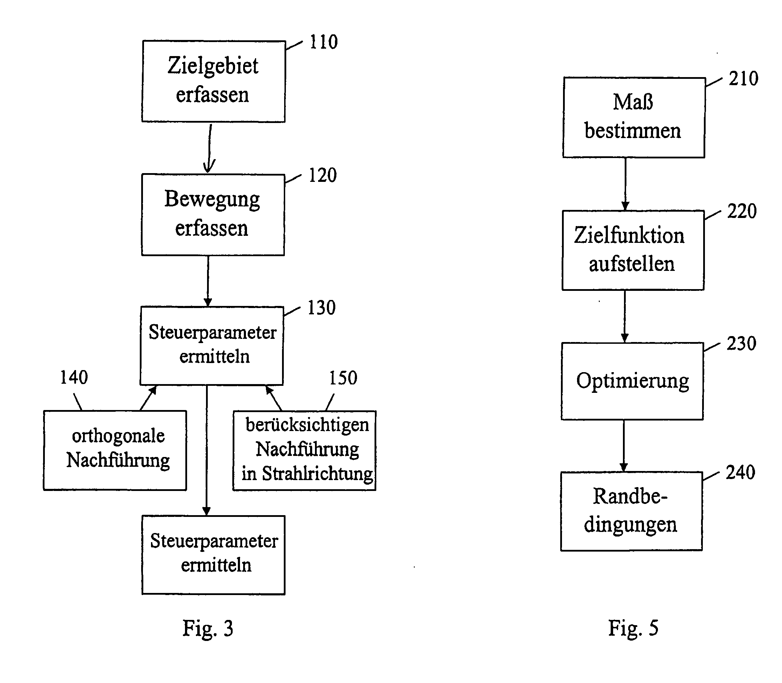

Die Bestrahlungsplanungsvorrichtung 68 umfasst eine Auswertungseinheit 70 zum Verarbeiten der Eingaben, und ist dabei derart ausgebildet, dass bei der Ermittlung der Steuerparameter ein Verfahren, wie es nachfolgend anhand von Figur 3 und Figur 4 näher erläutert wird, ausgeführt werden kann.

Die Steuerparameter werden der Bestrahlungsvorrichtung 10 weitergeleitet. Die Bestrahlungsvorrichtung 10 wird von einem Kontrollsystem gesteuert, das einzelne untergeordnete Steuerungseinrichtungen für verschiedene Teilsysteme umfasst. Hierzu gehören z.B. die Steuereinrichtung 66 für die Rasterscaneinrichtung 44, ggf. eine Steuereinrichtung 67 für die Energiemodulationsvorrichtung und weitere zusätzliche Steuereinrichtungen für andere Teile der Bestrahlungsvorrichtung 10, welche der Übersichtlichkeit halber nicht dargestellt sind. Weiterhin umfasst das Kontrollsystem Diagnoseeinrichtungen (auch diese der Übersichtlichkeit halber nicht dargestellt), mit welchen der Zustand von den verschiedenen Teilen der Bestrahlungsvorrichtung 10 überwacht werden kann. Das Kontrollsystem steuert dabei den Bestrahlungsverlauf entsprechend der ermittelten Steuerparameter.

Figur 3 zeigt als Ablaufdiagramm das erfindungsgemäße Verfahren. Das Verfahren dient der Bestimmung von Steuerparametern für eine Bestrahlungsanlage 10, in welcher eine Vielzahl von Strahlungsdosen nacheinander an unterschiedlichen Zielpunkten in einem Zielvolumen 56 appliziert werden können.

Im Verfahrensschritt 110 wird ein Zielgebiet bzw. Zielvolumen 56 erfasst. Im Verfahrensschritt 120 wird die Bewegung des Zielgebietes, bzw. des Zielvolumens 56, erfasst, z.B. mit Hilfe eines 4D-CT-Datensatzes. Die Erfassung des Zielgebiets/-volumens kann auch die Erfassung von weiteren, klinisch relevanten Gebieten wie zu schonende Organe (OAR für engl.: "organs at rist") umfassen.

Im verfahrensschritt 130 werden Steuerparameter zur Steuerung eines Strahls derart ermittelt, dass mit den Steuerparametern der Strahl der Bewegung des Zielgebiets nachgeführt werden und in dem Zielgebiet eine beispielsweise durch einen Anwender vordefinierte Dosisverteilung deponiert werden kann. In diesem Verfahrensschritt 130 wird der Satz der Steuerparameter, mit welchen eine anschließende Bestrahlung erfolgt, nicht notwendigerweise vollständig bestimmt. Dies bedeutet, dass bei dem Verfahrensschritt 130 auch nur ein Teil von Steuerparametern bestimmt werden kann, der zu einem späteren Zeitpunkt um weitere Steuerparameter ergänzt werden kann, welche dann in ihrer Gesamtheit einen kompletten, vollständigen Satz zum Steuern der Bestrahlungsanlage bilden. Gegebenenfalls kann der Satz zum Steuern der Bestrahlungsanlage auch online, d.h. während der Bestrahlung des Zielvolumens 56, ergänzt werden.

Hierbei wird in einer alternativen Ausführungsvariante im Schritt 140 bei der Ermittlung der Steuerparameter zumindest ein erster wählbarer Steuerparameter derart bestimmt, dass der Strahl der Bewegung des Zielgebiets bzw. Zielvolumens 56 lediglich orthogonal zur Strahlrichtung nachgeführt wird.

Dieser Steuerparameter kann dabei ein Parameter aus einer Vielzahl von Parametern sein, welche die Bestrahlung charakterisieren. Dieser Steuerparameter beschreibt dabei eine Größe, welche keinen unmittelbaren Zusammenhang mit der Bewegungsnachführung hat. Steuerparameter, die hierunter fallen können, werden anhand von Figur 4 näher beschrieben.

Falls es sich bei der Ermittlung der Steuerparameter herausstellt, dass selbst bei freier Wahl dieses Steuerparameters die Vorgabe nicht erfüllbar ist, lediglich ein orthogonales Tracking durchzuführen und dabei gleichzeitig die anderen Vorgaben wie die Deposition der Solldosisverteilung in ausreichender Qualität zu erfüllen, können gegebenenfalls ein oder mehrere weitere Steuerparameter als frei wählbar gesetzt werden. Durch die so geschaffenen zusätzlichen Freiheitsgrade ist es dann gegebenenfalls möglich, die Vorgaben der Solldosisverteilung und des lediglich orthogonalen Tracking zu erfüllen.

Alternativ werden in Schritt 150 bei der Ermittlung der Steuerparameter zumindest ein erster wählbarer Steuerparameter und ein weiterer Steuerparameter, welcher eine Energiemodulation des Strahls repräsentiert, ermittelt, wobei die Ermittlung des zumindest einen ersten Steuerparameters und des weiteren Steuerparameters unter Berücksichtigung der Bewegungsnachführung in Strahlrichtung erfolgt.

In dieser Ausführungsform wird folglich eine Bewegungsnachführung in Strahlrichtung zugelassen. Jedoch wird nicht nur der weitere Steuerparameter, welcher die Energiemodulation des Strahls repräsentiert und welcher damit unmittelbar in Zusammenhang mit der Bewegungsnachführung in Strahlrichtung steht, unter Berücksichtigung der Bewegungsnachführung in Strahlrichtung bestimmt, sondern auch der erste wählbarer Steuerparameter. Wie bei der Beschreibung des Verfahrenschritts 140 kann dieser Steuerparameter dabei ein Parameter aus einer Vielzahl von Parametern, welche die Bestrahlung charakterisieren, sein. Der erste wählbare Steuerparameter beschreibt dabei eine Größe, welche keinen unmittelbaren Zusammenhang mit der Bewegungsnachführung hat. Steuerparameter, die hierunter fallen können, werden anhand von Figur 4 näher beschrieben.

Diese Ausführungsform erlaubt es, Zielvorgaben hinsichtlich der Bewegungsnachführung in Strahlrichtung zu berücksichtigen. Beispielsweise kann eine obere Grenze gezogen werden, welche das Ausmaß der Bewegungsnachführung in Strahlrichtung beschreibt und welche nicht überschritten werden soll. Beispielsweise können der erste wählbarer Steuerparameter und der weitere Steuerparameter derart optimiert werden, dass die auftretende Bewegungsnachführung in Strahlrichtung reduziert bzw. gar minimiert ist, beispielsweise hinsichtlich der maximal erforderlichen Nachführung bzw. auch der erforderlichen Änderungen der Nachführung.

Falls sich bei der Ermittlung der Steuerparameter herausstellt, dass selbst bei freier Wahl des ersten wählbaren Steuerparameters die Vorgabe nicht erfüllbar ist, eine Zielvorgabe bezüglich der Strahlnachführung in Strahlrichtung einzuhalten und dabei gleichzeitig die anderen Vorgaben wie die Deposition der Solldosisverteilung in ausreichender Qualität zu erfüllen, können gegebenenfalls ein oder mehrere zusätzliche, weitere Steuerparameter als wählbar gesetzt werden. Durch die so geschaffenen zusätzlichen Freiheitsgrade ist es dann gegebenenfalls möglich, die Vorgaben, welche die Solldosisverteilung und die Bewegungsnachführung in Strahlrichtung betreffen, zu erfüllen.

Die in den Schritten 140 und 150 ermittelten Steuerparameter werden alternativ dem Schritt 130 als Eingabedaten zugeführt. Insgesamt wird so ein Satz von Steuerparametern erstellt, die - falls gewünscht - zur Steuerung eines Bestrahlungsverlauf in einem Verfahrenschritt 160 verwendet werden können.

In einer Ausgestaltung des Verfahrens kann in dem Verfahrensschritt 160 die Bestrahlung eines bewegten Zielgebietes erfolgen, wobei das Zielgebiet zumindest einen Teilbereich eines nicht-lebenden Körpers, insbesondere eines Phantoms, umfasst.

Figur 4 zeigt eine Auflistung von Steuerparametern, welche nicht in unmittelbarem Zusammenhang mit der Bewegungsnachführung stehen, welche aber im Rahmen der Bestrahlungsplanung derart gewählt werden, dass eine Vorgabe, welche die Bewegungsnachführung in Strahlrichtung betrifft, erfüllt wird.


Einstrahlrichtung:

Durch eine geeignete Wahl der Einstrahlrichtung des Partikelstrahls kann es möglich werden, Zielvorgaben, welche die Bewegungsnachführung in Strahlrichtung betreffen, einzuhalten, und trotzdem eine Solldosisverteilung in ausreichender Qualität zu deponieren.


Anzahl der Felder:

Das gleiche trifft zu, wenn die Anzahl der Felder nicht starr vorgegeben ist, sondern bei der Bestrahlungsplanung gewählt werden kann. Ein Feld ist durch eine Einstrahlrichtung gegeben. Wenn die Anzahl der Felder erhöht wird, d.h. wenn die Solldosisverteilung auf mehrere Felder mit unterschiedlichen Einstrahlrichtungen aufgeteilt wird, können Zielvorgaben besser eingehalten werden. Die Felder können dabei einzeln und/oder parallel betrachtet werden, d.h. dass die Steuerparameter für die einzelnen Felder getrennt oder gemeinsam ermittelt und insbesondere optimiert werden.

Überlapp der einzelnen Rasterpunkte:

Bei einem geringen Überlapp ist beispielsweise eine genaue Bewegungsnachführung notwendig. Bei einem größeren Überlapp kann eine Solldosis auch dann deponiert werden, wenn die Nachführung weniger genau durchgeführt, da eine bestimmte Vorgabe, welche die Bewegungsnachführung des Strahls in Strahlrichtung einschränkt, berücksichtigt werden muss. Der Nachteil eines größeren Überlapp ist, dass der Abfall der Dosisverteilung an den Rändern weniger steil ist. Je nach Struktur, welche das Zielvolumen umgibt, kann dies jedoch tolerabel sein.

Rescanning:

Unter Rescanning wird verstanden, dass ein Rasterpunkt mehrfach angefahren wird, bis eine gewünschte Dosis an dem Rasterpunkt deponiert wird. Ein Rescanning bewirkt oft, dass sich unerwünschte Effekte ausmitteln, welche vorgelegen hätten, wenn keine Rescanning durchgeführt worden wäre. Ohne Rescanning kann es zum Beispiel nicht möglich sein, eine Solldosisverteilung zu deponieren und gleichzeitig eine bestimmte Vorgabe bezüglich des Tracking in Strahlrichtung zu erfüllen. Wenn nun aber als Freiheitsgrad ein Rescanning zugelassen wird, kann es möglich werden, beide Vorgaben zu erfüllten. Dabei kann z.B. als wählbarer Parameter bestimmt werden, wie oft ein Rasterpunkt wiederholt angefahren wird, etc.

Einstellung des Scan-Pfades:

Wenn der Scan-Pfad starr vorgegeben ist, also die Reihenfolge, wie die Rasterpunkte abgetastet werden, kann dies beispielsweise dazu führen, dass eine bestimmte Vorgabe hinsichtlich der Bewegungsnachführung in Strahlrichtung nicht eingehalten werden kann. Dadurch, dass nun der Scan-Pfad geändert wird, kann eine Vorgabe bezüglich der Bewegungsnachführung wieder eingehalten werden. Insbesondere können dreidimensionale Scan-Pfade zugelassen werden, so dass nicht mehr Iso-Energieschicht nach Iso-Energieschicht hintereinander bestrahlt wird, sondern zwischen den Iso-Energieschichten auch dann gewechselt wird, bevor eine Iso-Energieschicht zu Ende bestrahlt worden ist. Dies schließt ein, dass beispielsweise die Iso-Energieschichten nicht mehr nacheinander, sondern zunächst alle geraden, dann alle ungeraden Iso-Energieschichten bestrahlt werden, oder ähnliche Ausgestaltungen.

Einstellung der Scan-Geschwindigkeit:

Wenn die Scan-Geschwindigkeit, die über die extrahierte Teilchenanzahl pro Zeiteinheit bzw. Strahlpuls festgelegt ist, starr vorgegeben ist, kann dies beispielsweise dazu führen, dass die Geschwindigkeit des Kompensationssystems und/oder die Änderung der Geschwindigkeit des Kompensationssystems nicht ausreicht, um den Strahl nachzuführen. Ist die Scan-Geschwindigkeit ein frei wählbarer Parameter, so kann - insbesondere durch eine Verringerung der Scan-Geschwindigkeit - die Anforderung an das Kompensationssystem reduziert werden. Bei Erhöhung der Scan-Geschwindigkeit kann beispielsweise die Anzahl der Rasterpunkte, die nacheinander ohne Änderung einer evtl. notwendigen longitudinalen Bewegungsnachführung bestrahlt werden können, erhöht werden. Die Änderung der Scan-Geschwindigkeit kann global pro Bestrahlungsvorgang, pro Strahlpuls oder gar für zumindest einen Rasterpunkt erfolgen.
Parameter, welcher eine Kombination verschiedener Arten von Bewegungsmanagement beschreibt:

Kombination aus Gating und Tracking:

Werden Rasterpunkte identifiziert, bei welchen sich eine bestimmte Vorgabe hinsichtlich der Bewegungsnachführung in Strahlrichtung nicht einhalten lässt, kann beispielsweise ein Zeitfenster bestimmt werden, während dem diese Rasterpunkte nicht bestrahlt werden sollen. Hierdurch lässt sich festlegen, dass diese Rasterpunkte lediglich dann bestrahlt werden, wenn die Bewegung des Zielvolumens nicht so stark ausgeprägt ist. Auf diese Weise kann eine Vorgabe hinsichtlich der Bewegungsnachführung in Strahlrichtung wieder erfüllt werden. Es wird folglich für diese Rasterpunkte ein Gating eingeführt. Ebenso kann für bestimmte Rasterpunkte, welche für die Dosisdeposition besonders wichtig sind, die Dosis im Gating-Verfahren appliziert werden, während bei Rasterpunkten, welche eine geringere Rolle für die Dosisdeposition spielen, ein Tracking - lediglich orthogonal oder auch in Strahlrichtung unter einer bestimmten Vorgabe - zugelassen werden.
Kombination aus lediglich orthogonalem Tracking und einem Tracking in Strahlrichtung:
Werden Rasterpunkte identifiziert, bei welchen eine Solldosisverteilung nicht zu erreichen ist, wenn lediglich ein Tracking in orthogonaler Richtung durchgeführt wird, können diese Rasterpunkte für ein Tracking in z-Richtung freigegeben werden.
Fig. 5 zeigt schematisch die Verfahrensschritte, die bei der Bestimmung der wählbaren Steuerparameter, welche anhand von Fig. 4 näher erläutert wurden, durchgeführt werden können.

In einem ersten Schritt kann ein Maß bestimmt werden, das die Bewegungsnachführung in Strahlrichtung berücksichtigt (Verfahrenschritt 210).

Dieses Maß berücksichtigt die Ausprägung der Steuerparameter, welche bei einer durchzuführenden Bewegungsnachführung in Strahlrichtung auftreten. Das Maß kann z.B. eine Amplitude der Bewegungsnachführung des Strahls in Strahlrichtung, eine Geschwindigkeit der Bewegungsnachführung des Strahls in Strahlrichtung, und/oder eine Variation der Geschwindigkeit der Bewegungsnachführung des Strahls in Strahlrichtung kennzeichnen.

Die Bestimmung des Maßes bietet eine einfache Möglichkeit, die Bewegungsnachführung in Strahlrichtung bei der Bestimmung der Steuerparameter zu berücksichtigen.

Z.B. kann eine Zielfunktion bereitgestellt werden, in welche das Maß einfließt (Verfahrenschritt 220).

Anschließend werden die Steuerparameter aufgrund der Zielfunktion optimiert (Verfahrenschritt 230). Diese Optimierung kann unter Berücksichtigung von Randbedingungen erfolgen, welche die Vorgaben, die an einen Bestrahlungsplan gestellt werden, widerspiegeln (Verfahrenschritt 240).

Im Folgenden wird beispielhaft eine Formel angegeben, welche die Zielfunktion beschreibt, die es zu optimieren gilt.

Ein zu bestrahlendes Zielvolumen ist in einem Zielgebiet definiert. Dies kann z.B. mithilfe eines CT-Datensatzes erfolgen. Dieses Zielgebiet bzw. dieses Zielvolumen liegt der Zielfunktion zu Grunde.

Das Zielgebiet kann über eine Vielzahl von Voxeln M' beschrieben werden. Der Index k indiziert die einzelnen Voxel k = 1 ... M'. In dem Zielgebiet kann eine Solldosis $D_{pre}^{k}$ vorgegeben werden.

Das Zielvolumen in dem Zielgebiet kann über eine Anzahl M von Rasterpunkten, die nacheinander angefahren werden, beschrieben werden. Die Rasterpunkte sind mit i indiziert, i = 1 ... M.

Die Bewegung des Zielvolumens kann über eine Anzahl L von 4D-CT-Phasen beschrieben werden, also über ein vierdimensionales CT-Bild, in welchem die Bewegung abgebildet ist. Die einzelnen Phasen sind mit dem Index j, j= 1 ... L indiziert.

In die Zielfunktion fließen als zu optimierende Parameter die Anzahl der Teilchen pro Rasterpunkt $\overline{N}$ = ($N_1$, $N_2$, ...$N_M$), die Fluenz pro Rasterpunkt $\overline{I}$ = ($I_1$, $I_2$, ...$I_M$) und der Scanpfad $S$ = ($S_1$, $S_2$, ...$S_M$) ein. Die Fluenz ist dabei ein indirektes Maß dafür, wie schnell oder langsam ein Rasterpunkt i bestrahlt wird. Der Scanpfad gibt die Reihenfolge an, in der die einzelnen Rasterpunkte angefahren werden.

Aus dem Scanpfad $\overline{S}$ lässt sich beispielsweise die Größe $S_i$- $S_{i-1}$ = | $(x, y, z)_i$ - $(x, y, z)_{i-1}$| berechnen, welche die räumliche Distanz zwischen zwei aufeinander folgenden Rasterpunkten angibt.

Die Zielfunktion $\chi^2$ lautet:

$$\chi^2(\vec{N}, \vec{I}, \vec{S}) = \sum_k \left( \frac{D_{pre}^k - D_{act}^k(\vec{N})}{\Delta D_{pre}^k} \right)^2 \qquad (1.1)$$

$$+ \sum_{i,j} a_i N_i^2 \left( \frac{\delta z^{ij}}{\Delta \delta z^i} \right)^2 \qquad (1.2)$$

$$+ \sum_{i,j} b_i \left( \frac{\frac{d(\delta z)^{ij}}{dt}(\vec{I}, \vec{S})}{\Delta(\frac{d(\delta z)}{dt})^i} \right)^2 \qquad (1.3)$$

$$+ \sum_{i,j} \frac{c_i}{(I^i - I_{min})^2} \qquad (1.4)$$

$$+ \sum_{i>2} d_i \left( \frac{S_i - S_{i-1}}{\Delta S^i} \right)^2 \cdot \theta \left( \left| S_i - S_{i-1} \right| - S_{norm} \right) \qquad (1.5)$$

mit folgenden Randbedingungen für alle i = 1 ... M:

$$N_i \geq N_{\min}$$

$$I_i \geq I_{\min}$$

$$\Delta \delta z^i = f_{1i} \cdot \delta z_{\max}, \quad f_{1i} < 1$$

$$\Delta \left( \frac{d(\delta z)}{dt} \right)^i = f_{2i} \cdot \left( \frac{d(\delta z)}{dt} \right)_{\max}, \quad f_{2i} < 1$$

$$\Delta S^i = f_{3i} \cdot S_{norm}, \quad f_{3i} < 1$$

Die Wichtungen $a_i$, $b_i$, $c_i$, $d_i$ bestimmen die Bedeutung der einzelnen Summanden.

Der erste Summand der Formel beschreibt die Abweichung der aktuellen Dosis $D_{act}^k(\vec{N})$ von der Solldosis $D_{pre}^k$. Mit dem Faktor $\Delta D_{pre}^k$ kann die Abweichung voxelweise mehr oder weniger stark gewichtet werden.

Der zweite Summand der Formel berücksichtigt die Bewegungsnachführung $\delta z^{ij}$ des Strahls in z-Richtung. $\delta z^{ij}$ gibt an, wie groß die Änderung in z-Richtung für einen Rasterpunkt i beim Übergang von der Referenzphase zzur Phase j ist. Mit dem Faktor $\Delta \delta z^i$ können diese Änderungen pro Rasterpunkt gewichtet werden. $\delta z_{max}$ ist dabei die maximale Änderung, die noch tolerabel ist. Hiermit können beispielsweise Einschränkungen, die durch das System bzw. dessen Auslegung gegeben sind, berücksichtigt werden.

Der dritte Summand der Formel berücksichtigt die Geschwindigkeit der Bewegungsnachführung $\frac{d(\delta z)^{ij}}{dt}$ des Strahls in z-Richtung pro Rasterpunkt und Bewegungsphase. Diese Geschwindigkeitsänderung $\frac{d(\delta z)^{ij}}{dt}(\vec{I}, \vec{S})$ hängt von der Fluenz und von dem Strahlpfad ab. Mit dem Faktor $\Delta \left( \frac{d(\delta z)}{dt} \right)^i$ können diese Änderungen pro Rasterpunkt gewichtet werden. $\left( \frac{d(\delta z)}{dt} \right)_{\max}$ ist dabei die maximale Geschwindigkeitsänderung, die noch tolerabel ist. Hiermit können beispielsweise Einschränkungen, die durch das System bzw. dessen Auslegung gegeben sind, berücksichtigt werden.

Der vierte Summand der Formel berücksichtigt, dass bei der Optimierung die Fluenz pro Rasterpunkt nicht zu gering wird, was die Dauer der Bestrahlung insgesamt verlängern würde.

Der fünfte Summand der Formel berücksichtigt, dass bei der Wahl des Strahlpfades die Abstände der einzelnen Rasterpunkte zueinander nicht zu groß werden. Mit dem Faktor $\Delta S^i$ können Rasterpunkte unterschiedlich gewichtet werden. Durch die $\theta$-Funktion ist jedoch gewährleistet, dass der Abstand der Rasterpunkte beliebig gewählt werden kann, solange er sich unterhalb eines Schwellenwertes $S_{norm}$ befindet.

Anhand der obigen Gleichung wurde also gezeigt, wie beispielsweise der Strahlpfad, die Anzahl der zu deponierenden Teilchen und die Fluenz in einer Zielfunktion berücksichtigt werden können. Mit der Zielfunktion wird ebenso die Bewegungsnachführung des Partikelstrahls in Strahlrichtung berücksichtigt, sodass durch die optimierten Parameter gewährleistet ist, dass die Bewegungsnachführung auch ausgeführt werden kann.

Analoge Zielfunktionen können auch dann verwendet werden, wenn andere Parameter als die Strahlnachführung, die Fluenz und die Anzahl der Teilchen hinsichtlich einer Strahlnachführung in z-Richtung optimiert werden sollen.

**Patentansprüche**

1. vorrichtung zur Bestimmung von Steuerparametern für eine Bestrahlungsanlage, mit welcher eine Vielzahl von Strahlungsdosen nacheinander an unterschiedlichen Zielpunkten in einem Zielvolumen deponierbar ist, umfassend:

   eine Eingabeeinrichtung, welche zum Erfassen eines Zielgebietes und zum Erfassen einer Bewegung des Zielgebiets ausgebildet ist,
   eine Auswertungseinrichtung zum Ermitteln von Steuerparametern zur Steuerung eines Strahls der Bestrahlungsanlage, derart, dass mit Hilfe der Steuerparameter ein Strahl der Bewegung des Zielgebiets nachführbar ist und in dem Zielgebiet eine definierte Dosisverteilung deponierbar ist,
   **gekennzeichnet dadurch, dass**
   die Auswertungseinrichtung dazu ausgelegt ist, zumindest einen ersten wählbaren der folgend definierten Steuerparameter zu ermitteln:

   - Einstrahlrichtung des Strahls relativ zum Zielgebiet,
   - Anzahl von Feldern im Zielvolumen,
   - Überlapp einzelner Rasterpunkte des Zielvolumens,
   - Rescanning und/oder Anzahl der Rescanning-Durchgänge,
   - Einstellung des Scan-Pfades des Strahls im Zielvolumen,
   - Kombination aus Gating und Tracking,
   - Kombination aus lediglich orthogonalem Tracking und einem Tracking in Strahlrichtung,
   - Einstellung einer Scangeschwindigkeit, und
   wobei die Auswertungseinrichtung dazu ausgelegt ist, einen weiteren, eine Energiemodulation des Strahls repräsentierenden Steuerparameter zu ermitteln, wobei die Bewegungsnachführung in Strahlrichtung berücksichtigt ist, um anhand des ersten und des weiteren Steuerparameters die übrigen wählbaren Steuerparameter zu ermitteln, die nicht unmittelbar mit der Energiemodulation des Strahls oder unmittelbar mit der Strahlnachführung in lateraler Richtung zusammenhängen,
   wobei bei der Ermittlung der übrigen wählbaren Steuerparameter ein Maß genutzt wird, welches die durchzuführende Bewegungsnachführung in Strahlrichtung berücksichtigt.

2. Vorrichtung nach Anspruch 1,
   wobei die Auswertungseinrichtung derart ausgebildet ist, dass die Ermittlung der Steuerparameter derart erfolgt, dass der Strahl der Bewegung des Zielgebiets in zumindest zwei unterschiedlichen Richtungen nachgeführt wird.

3. Vorrichtung nach einem der Ansprüche 1 oder 2,
   wobei das Maß eine Amplitude der Bewegungsnachführung des Strahls in Strahlrichtung, eine Geschwindigkeit der Bewegungsnachführung des Strahls in Strahlrichtung, und/oder eine Variation der Geschwindigkeit der Bewegungsnachführung des Strahls in Strahlrichtung berücksichtigt.

4. Vorrichtung nach einem der Ansprüche 1 bis 3,
   wobei die Auswertungseinrichtung derart ausgebildet ist, dass das Maß in eine Zielfunktion einfließt in der eine Bewegungsnachführung des Strahls in Strahlrichtung bestraft ist und deren Auswertung, insb. Optimierung, zur Ermittlung der Steuerparameter beiträgt.

**5.** Vorrichtung nach einem der Ansprüche 1 bis 4,
wobei die Auswertungseinrichtung derart ausgebildet ist, dass der zumindest eine erste Steuerparameter zumindest eine Bewegungsphase bestimmt, während der wenigstens ein erster Teil der Zielpunkte nicht angesteuert wird.

**6.** Vorrichtung nach einem der Ansprüche 1 bis 5,
wobei die Auswertungseinrichtung derart ausgebildet ist, dass der zumindest eine erste Steuerparameter einen zweiten Teil der Zielpunkte bestimmt, der ohne Durchführen einer Bewegungsnachführung des Strahls angesteuert wird.

**7.** Vorrichtung nach einem der Ansprüche 1 bis 6,
wobei die Auswertungseinrichtung derart ausgebildet ist, dass der zumindest eine erste Steuerparameter ein wiederholtes Anfahren der Zielpunkte und/oder eine Reihenfolge des Anfahrens der Zielpunkte bestimmt.

**8.** Vorrichtung nach einem der Ansprüche 1 bis 7,
wobei die Auswertungseinrichtung derart ausgebildet ist, dass der zumindest eine erste Steuerparameter eine räumliche Orientierung einer Einstrahlrichtung, eine Anzahl von Einstrahlrichtungen und/oder einen Überlapp von Zielpunkten bestimmt.

**9.** Bestrahlungsanlage mit einer Vorrichtung zur Bestimmung von Steuerparametern nach einem der Ansprüche 1 bis 8.

**10.** Verfahren zum Bestimmen von Steuerparametern für eine Bestrahlungsanlage, in welcher eine Vielzahl von Strahlungsdosen nacheinander an unterschiedlichen Zielpunkten in einem Zielvolumen deponiert werden können, aufweisend folgende Schritte:

Erfassen eines Zielgebietes mit einer Eingabeeinrichtung,
Erfassen einer Bewegung des Zielgebiets mit der Eingabeeinrichtung,
Ermitteln von Steuerparametern im Vorfeld einer Bestrahlung zur Steuerung eines Strahls der Bestrahlungsanlage, derart, dass mit den Steuerparametern der Strahl der Bewegung des Zielgebiets nachgeführt werden und in dem Zielgebiet eine definierte Dosisverteilung deponiert werden kann,
**gekennzeichnet dadurch, dass**
bei der Ermittlung der Steuerparameter durch die Auswertungseinrichtung zumindest ein erster wählbarer der folgend definierten Steuerparameter ermittelt wird:

- Einstrahlrichtung des Strahls relativ zum Zielgebiet,
- Anzahl von Feldern im Zielvolumen,
- Überlapp einzelner Rasterpunkte des Zielvolumens,
- Rescanning und/oder Anzahl der Rescanning-Durchgänge,
- Einstellung des Scan-Pfades des Strahls im Zielvolumen,
- Kombination aus Gating und Tracking,
- Kombination aus lediglich orthogonalem Tracking und einem Tracking in Strahlrichtung,
- Einstellung einer Scangeschwindigkeit, und wobei bei der Ermittlung der Steuerparameter durch die Auswertungseinrichtung zumindest ein weiterer eine Energiemodulation des Strahls repräsentierender Steuerparameter ermittelt wird, wobei die Bewegungsnachführung in Strahlrichtung berücksichtigt wird, um anhand des ersten und des weiteren Steuerparameters die übrigen wählbaren Steuerparameter zu ermitteln, die nicht unmittelbar mit der Energiemodulation des Strahls oder unmittelbar mit der Strahlnachführung in lateraler Richtung zusammenhängen

wobei bei der Ermittlung der übrigen wählbaren Steuerparameter ein Maß genutzt wird, welches die durchzuführende Bewegungsnachführung in Strahlrichtung berücksichtigt.

**11.** Verfahren zur Bestrahlung eines bewegten Zielgebiets mit einem Satz von Steuerparametern zur Steuerung einer Bestrahlungseinrichtung, wobei die Steuerparameter mit einem Verfahren nach Anspruch 10 ermittelt worden sind und wobei das Zielvolumen zumindest einen Teilbereich eines nicht-lebenden Körpers, insbesondere eines Phantoms, zur Überprüfung einer Bestrahlungsplanung umfasst.

**Claims**

1. Apparatus for determining control parameters for an irradiation unit, with which a large number of radiation doses is depositable in succession at different target points within a target volume, comprising:

   an input device, which is configured to capture a target region and to capture a movement of the target region, an evaluation device for ascertaining control parameters for controlling a beam from the irradiation unit such that a beam can track the movement of the target region using the control parameters, and a defined dose distribution is depositable in the target region,
   **characterized in that**
   the evaluation device is configured to ascertain at least one first selectable control parameter from the following defined control parameters:

   - irradiation direction of the beam relative to the target region,
   - number of fields in the target volume,
   - overlap of individual raster points of the target volume,
   - rescanning and/or number of rescanning cycles,
   - setting of the scanning path of the beam in the target volume,
   - combination of gating and tracking,
   - combination of merely orthogonal tracking and tracking in the beam direction,
   - setting of a scanning speed, and

   wherein the evaluation device is configured to ascertain a further control parameter representing an energy modulation of the beam, wherein the movement tracking in the beam direction is taken into account in order to ascertain, on the basis of the first and of the further control parameters, the remaining selectable control parameters which are not directly associated with the energy modulation of the beam or directly with the beam tracking in the lateral direction,
   wherein, in the ascertainment of the remaining selectable control parameters, a measure is used which takes into account the movement tracking in the beam direction to be carried out.

2. Apparatus according to Claim 1, wherein the evaluation device is configured such that the control parameters are ascertained such that the beam tracks the movement of the target region in at least two different directions.

3. Apparatus according to one of Claims 1 and 2, wherein the measure takes into account an amplitude of the movement tracking of the beam in the beam direction, a speed of the movement tracking of the beam in the beam direction, and/or a variation in the speed of the movement tracking of the beam in the beam direction.

4. Apparatus according to one of Claims 1 to 3, wherein the evaluation device is configured such that the measure is incorporated in a target function in which movement tracking of the beam in the beam direction is punished and the evaluation thereof, in particular optimization, contributes to the ascertainment of the control parameters.

5. Apparatus according to one of Claims 1 to 4, wherein the evaluation device is configured such that the at least one first control parameter determines at least one movement phase, during which at least a first part of the target points is not targeted.

6. Apparatus according to one of Claims 1 to 5, wherein the evaluation device is configured such that the at least one first control parameter determines a second part of the target points, which is targeted without movement tracking of the beam being carried out.

7. Apparatus according to one of Claims 1 to 6, wherein the evaluation device is configured such that the at least one first control parameter determines repeat targeting of the target points and/or a sequence of the targeting of the target points.

8. Apparatus according to one of Claims 1 to 7, wherein the evaluation device is configured such that the at least one first control parameter determines a spatial orientation of an irradiation direction, a number of irradiation directions and/or an overlap of target points.

9. Irradiation unit having an apparatus for determining control parameters according to one of Claims 1 to 8.

**10.** Method for determining control parameters for an irradiation unit in which a large number of radiation doses can be deposited in succession at different target points within a target volume, having the following steps:

capturing a target region using an input device,
capturing a movement of the target region using the input device,
ascertaining control parameters prior to irradiation for controlling a beam from the irradiation unit such that the beam can track the movement of the target region using the control parameters, and a defined dose distribution can be deposited in the target region,
**characterized in that**,
in the ascertainment of the control parameters by way of the evaluation device, at least one first selectable control parameter from the following defined control parameters is ascertained:

- irradiation direction of the beam relative to the target region,
- number of fields in the target volume,
- overlap of individual raster points of the target volume,
- rescanning and/or number of rescanning cycles,
- setting of the scanning path of the beam in the target volume,
- combination of gating and tracking,
- combination of merely orthogonal tracking and tracking in the beam direction,
- setting of a scanning speed, and

wherein, in the ascertainment of the control parameters by way of the evaluation device, at least one further control parameter representing an energy modulation of the beam is ascertained, wherein the movement tracking in the beam direction is taken into account in order to ascertain, on the basis of the first and of the further control parameters, the remaining selectable control parameters which are not directly associated with the energy modulation of the beam or directly with the beam tracking in the lateral direction,
wherein, in the ascertainment of the remaining selectable control parameters, a measure is used which takes into account the movement tracking in the beam direction to be carried out.

**11.** Method for irradiating a moved target region using a set of control parameters for controlling an irradiation unit, wherein the control parameters have been ascertained using a method according to Claim 10 and wherein the target volume comprises at least one partial region of a non-living body, in particular of a phantom, for checking an irradiation plan.

**Revendications**

**1.** Dispositif pour déterminer des paramètres de commande pour une installation de radiothérapie qui permet d'appliquer une pluralité de doses de rayonnement successives sur différents points cibles d'un volume cible, comprenant :

un dispositif d'entrée qui est conçu pour détecter une zone cible et pour détecter un mouvement de la zone cible,
un dispositif d'évaluation pour déterminer des paramètres de commande afin de commander un faisceau de l'installation de radiothérapie de façon qu'un faisceau puisse suivre le mouvement de la zone cible à l'aide des paramètres de commande et qu'une répartition de doses définie puisse être appliquée dans la zone cible,
**caractérisé en ce que**
le dispositif d'évaluation est conçu pour déterminer au moins un premier paramètre de commande sélectionnable parmi les paramètres de commande définis ci-après :

- direction de rayonnement du faisceau par rapport à la zone cible,
- nombre de champs dans le volume cible,
- chevauchement de certains points de trame du volume cible,
- rebalayage et/ou nombre de passes de rebalayage,
- réglage du chemin de balayage du faisceau dans le volume cible,
- combinaison de gating et de tracking,
- combinaison d'un tracking seulement orthogonal et d'un tracking dans la direction du faisceau,
- réglage d'une vitesse de balayage,

et le dispositif d'évaluation étant conçu pour déterminer un autre paramètre de commande représentant une modulation d'énergie du faisceau, le suivi de mouvement dans la direction du faisceau étant pris en compte pour déterminer, à l'aide du premier et de l'autre paramètre de commande, les paramètres de commande sélectionnables restants qui ne sont pas directement en rapport avec la modulation d'énergie du faisceau ou sont directement en rapport avec le suivi de faisceau dans la direction latérale,

un critère étant utilisé lors de la détermination des paramètres de commande sélectionnables restants, lequel tient compte du suivi de mouvement à effectuer dans la direction du faisceau.

2. Dispositif selon la revendication 1,
   dans lequel le dispositif d'évaluation est conçu de façon que la détermination des paramètres de commande ait lieu de façon que le faisceau suive le mouvement de la zone cible dans au moins deux directions différentes.

3. Dispositif selon une des revendications 1 ou 2,
   dans lequel le critère tient compte d'une amplitude du suivi de mouvement du faisceau dans la direction du faisceau, d'une vitesse du suivi de mouvement du faisceau dans la direction du faisceau et/ou d'une variation de la vitesse du suivi de mouvement du faisceau dans la direction du faisceau.

4. Dispositif selon une des revendications 1 à 3,
   dans lequel le dispositif d'évaluation est conçu de façon que le critère soit pris en compte dans une fonction cible dans laquelle un suivi de mouvement du faisceau dans la direction du faisceau est pénalisé et dont l'évaluation, en particulier l'optimisation, contribue à la détermination des paramètres de commande.

5. Dispositif selon une des revendications 1 à 4,
   dans lequel le dispositif d'évaluation est conçu de façon que ledit au moins un premier paramètre de commande détermine au moins une phase de mouvement pendant laquelle au moins une première partie des points cibles n'est pas atteinte.

6. Dispositif selon une des revendications 1 à 5,
   dans lequel le dispositif d'évaluation est conçu de façon que ledit au moins un premier paramètre de commande détermine une deuxième partie des points cibles qui est atteinte sans effectuer un suivi de mouvement du faisceau.

7. Dispositif selon une des revendications 1 à 6,
   dans lequel le dispositif d'évaluation est conçu de façon que ledit au moins un premier paramètre de commande détermine une atteinte répétée des points cibles et/ou un ordre d'atteinte des points cibles.

8. Dispositif selon une des revendications 1 à 7,
   dans lequel le dispositif d'évaluation est conçu de façon que ledit au moins un premier paramètre de commande détermine une orientation spatiale d'une direction de rayonnement, un nombre de directions de rayonnement et/ou un chevauchement de points cibles.

9. Installation de radiothérapie avec un dispositif pour déterminer des paramètres de commande selon une des revendications 1 à 8.

10. Procédé pour déterminer des paramètres de commande pour une installation de radiothérapie qui permet d'appliquer une pluralité de doses de rayonnement successives sur différents points cibles d'un volume cible, présentant les étapes consistant à :

    détecter une zone cible avec un dispositif d'entrée,
    détecter un mouvement de la zone cible avec le dispositif d'entrée,
    déterminer des paramètres de commande avant une radiothérapie afin de commander un faisceau de l'installation de radiothérapie, de façon qu'un faisceau puisse suivre le mouvement de la zone cible à l'aide des paramètres de commande et qu'une répartition de doses définie puisse être appliquée dans la zone cible,
    **caractérisé en ce que**
    lors de la détermination des paramètres de commande par le dispositif d'évaluation, au moins un premier paramètre de commande sélectionnable parmi les paramètres de commande définis ci-après est déterminé :

      - direction de rayonnement du faisceau par rapport à la zone cible,
      - nombre de champs dans le volume cible,
      - chevauchement de certains points de trame du volume cible,

- rebalayage et/ou nombre de passes de rebalayage,
- réglage du chemin de balayage du faisceau dans le volume cible,
- combinaison de gating et de tracking,
- combinaison d'un tracking seulement orthogonal et d'un tracking dans la direction du faisceau,
- réglage d'une vitesse de balayage,

et au moins un autre paramètre de commande, représentant une modulation d'énergie du faisceau, étant déterminé lors de la détermination des paramètres de commande par le dispositif d'évaluation, le suivi de mouvement dans la direction du faisceau étant pris en compte pour déterminer, à l'aide du premier et de l'autre paramètre de commande, les paramètres de commande sélectionnables restants qui ne sont pas directement en rapport avec la modulation d'énergie du faisceau ou sont directement en rapport avec le suivi de faisceau dans la direction latérale,

un critère étant utilisé lors de la détermination des paramètres de commande sélectionnables restants, lequel tient compte du suivi de mouvement à effectuer dans la direction du faisceau.

11. Procédé de radiothérapie d'une zone cible en mouvement avec un jeu de paramètres de commande destiné à commander un dispositif de radiothérapie, les paramètres de commande ayant été déterminés avec un procédé selon la revendication 10, et le volume cible comprenant au moins une zone partielle d'un corps non vivant, en particulier d'un fantôme, pour vérifier un plan de radiothérapie.

Fig. 1

Fig. 2

Einstrahlrichtung

Anzahl der Felder

Überlapp der einzelnen Rasterpunkte

Rescanning

Einstellung des Scan-Pfades

Kombination aus Gating und Tracking

Kombination aus lediglich orthogonalem Tracking
und einem Tracking in Strahlrichtung

Einstellung der Scangeschwindigkeit

Fig. 4

EP 2 352 555 B1

Fig. 5

Fig. 3

**EP 2 352 555 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6891177 B1
- US 6710362 B2
- US 20060033042 A1
- WO 0207817 A2
- WO 2007079854 A2